# EUROPEAN PATENT SPECIFICATION

(11) **EP 3 017 041 B1**
(45) Date of publication and mention of the grant of the patent: **06.11.2019**
(21) Application number: 14735569.7
(22) Date of filing: 03.07.2014
(51) Int. Cl.: C12N 9/10

(54) **N-TERMINALLY TRUNCATED GLYCOSYLTRANSFERASES**
N-TERMINAL VERKÜRZTE GLYKOSYLTRANSFERASEN
GLYCOSYLTRANSFÉRASES TRONQUÉS À L'EXTRÉMITÉ N

(30) Priority: 05.07.2013 EP 13175349
(43) Date of publication of application: 11.05.2016
(73) Proprietor: F. Hoffmann-La Roche AG, 4070 Basel (CH); Roche Diagnostics GmbH, 68305 Mannheim (DE)
(72) Inventor: CZABANY, Tibor, 3430 Tulln an der Donau (AT); ENGEL, Alfred, 82362 Weilheim (DE); GREIF, Michael, 82377 Penzberg (DE); JUNG, Christine, 82393 Iffeldorf (DE); LULEY, Christiane, A-8063 Hoef-Praebach (AT); MALIK, Sebastian, 82387 Antdorf (DE); MUELLER, Rainer, 82377 Penzberg (DE); NIDETZKY, Bernd, A-8010 Graz (AT); RIBITSCH, Doris, A-8042 Graz (AT); SCHMOELZER, Katharina, A-8010 Graz (AT); SCHWAB, Helmut, A-8043 Graz (AT); SOBEK, Harald, 88400 Biberach (DE); SUPPMANN, Bernhard, 82362 Weilheim (DE); THOMANN, Marco, 82547 Beuerberg (DE); ZITZENBACHER, Sabine, A-8010 Graz (AT)
(74) Representative: Burger, Alexander
(86) International application number: PCT/EP2014/064214
(87) International publication number: WO 2015/001034

(56) References cited:
- WO-A2-2007/135194
- SHINOBU KITAZUME-KAWAGUCHI ET AL: "The relationship between ST6Gal I Golgi retention and its cleavage-secretion", GLYCOBIOLOGY, vol. 9, 1 January 1999 (1999-01-01), pages 1397-1406, XP055087331,
- SANDRINE DONADIO ET AL: "Recognition of cell surface acceptors by two human [alpha]-2,6-sialyltransferases produced in CHO cells", BIOCHIMIE, vol. 85, no. 3-4, 1 March 2003 (2003-03-01), pages 311-321, XP055087254, ISSN: 0300-9084, DOI: 10.1016/S0300-9084(03)00080-4
- LEGAIGNEUR P ET AL: "Exploring the acceptor substrate recognition of the human beta-Galactoside alpha2,6-sialyltransferase", JOURNAL OF BIOLOGICAL CHEMISTRY, AMERICAN SOCIETY FOR BIOCHEMISTRY AND MOLECULAR BIOLOGY, US, vol. 276, no. 24, 15 June 2001 (2001-06-15), pages 21608-21617, XP002326446, ISSN: 0021-9258, DOI: 10.1074/JBC.M100860200
- CHEN CHUN ET AL: "Minimal structural and glycosylation requirements for ST6Gal I activity and trafficking", GLYCOBIOLOGY, OXFORD UNIVERSITY PRESS, US, vol. 10, no. 5, 1 May 2000 (2000-05-01), pages 531-583, XP002467896, ISSN: 0959-6658, DOI: 10.1093/GLYCOB/10.5.531

### SEQUENCE LISTING

<110> F. Hoffmann-La Roche AG, Basel (CH) Roche Diagnostics GmbH, Mannheim (DE)
<120> N-terminally truncated glycosyltransferases
<130> 31663 WO
<150> EP13175349.3
   <151> 2013-07-05
<160> 19
<170> PatentIn version 3.5
<210> 1
   <211> 406
   <212> PRT
   <213> Homo sapiens
<220>
   <221> MISC_FEATURE
   <223> hST6-Gal-I WT polypeptide
<400> 1
   Met Ile His Thr Asn Leu Lys Lys Lys Phe Ser Cys Cys Val Leu Val
   1               5                   10                  15
   Phe Leu Leu Phe Ala Val Ile Cys Val Trp Lys Glu Lys Lys Lys Gly
               20                  25                  30
   Ser Tyr Tyr Asp Ser Phe Lys Leu Gln Thr Lys Glu Phe Gln Val Leu
           35                  40                  45
   Lys Ser Leu Gly Lys Leu Ala Met Gly Ser Asp Ser Gln Ser Val Ser
       50                  55                  60
   Ser Ser Ser Thr Gln Asp Pro His Arg Gly Arg Gln Thr Leu Gly Ser
   65                  70                  75                  80
   Leu Arg Gly Leu Ala Lys Ala Lys Pro Glu Ala Ser Phe Gln Val Trp
                   85                  90                  95
   Asn Lys Asp Ser Ser Ser Lys Asn Leu Ile Pro Arg Leu Gln Lys Ile
               100                 105                 110
   Trp Lys Asn Tyr Leu Ser Met Asn Lys Tyr Lys Val Ser Tyr Lys Gly
           115                 120                 125
   Pro Gly Pro Gly Ile Lys Phe Ser Ala Glu Ala Leu Arg Cys His Leu
       130                 135                 140
   Arg Asp His Val Asn Val Ser Met Val Glu Val Thr Asp Phe Pro Phe
   145                 150                 155                 160
   Asn Thr Ser Glu Trp Glu Gly Tyr Leu Pro Lys Glu Ser Ile Arg Thr
                   165                 170                 175
   Lys Ala Gly Pro Trp Gly Arg Cys Ala Val Val Ser Ser Ala Gly Ser
               180                 185                 190
   Leu Lys Ser Ser Gln Leu Gly Arg Glu Ile Asp Asp His Asp Ala Val
           195                 200                 205
   Leu Arg Phe Asn Gly Ala Pro Thr Ala Asn Phe Gln Gln Asp Val Gly
       210                 215                 220
   Thr Lys Thr Thr Ile Arg Leu Met Asn Ser Gln Leu Val Thr Thr Glu
   225                 230                 235                 240
   Lys Arg Phe Leu Lys Asp Ser Leu Tyr Asn Glu Gly Ile Leu Ile Val
                   245                 250                 255
   Trp Asp Pro Ser Val Tyr His Ser Asp Ile Pro Lys Trp Tyr Gln Asn
               260                 265                 270
   Pro Asp Tyr Asn Phe Phe Asn Asn Tyr Lys Thr Tyr Arg Lys Leu His
           275                 280                 285
   Pro Asn Gln Pro Phe Tyr Ile Leu Lys Pro Gln Met Pro Trp Glu Leu
       290                 295                 300
   Trp Asp Ile Leu Gln Glu Ile Ser Pro Glu Glu Ile Gln Pro Asn Pro
   305                 310                 315                 320
   Pro Ser Ser Gly Met Leu Gly Ile Ile Ile Met Met Thr Leu Cys Asp
                   325                 330                 335
   Gln Val Asp Ile Tyr Glu Phe Leu Pro Ser Lys Arg Lys Thr Asp Val
               340                 345                 350
   Cys Tyr Tyr Tyr Gln Lys Phe Phe Asp Ser Ala Cys Thr Met Gly Ala
           355                 360                 365
   Tyr His Pro Leu Leu Tyr Glu Lys Asn Leu Val Lys His Leu Asn Gln
       370                 375                 380
   Gly Thr Asp Glu Asp Ile Tyr Leu Leu Gly Lys Ala Thr Leu Pro Gly
   385                 390                 395                 400
   Phe Arg Thr Ile His Cys
                   405
<210> 2
   <211> 7
   <212> PRT
   <213> Artificial Sequence
<220>
   <223> consensus sequence
<220>
   <221> misc_feature
   <222> (4)..(4)
   <223> Xaa can be any naturally occurring amino acid
<400> 2
   Gln Val Trp Xaa Lys Asp Ser
   1               5
<210> 3
   <211> 379
   <212> PRT
   <213> Artificial Sequence
<220>
   <223> delta27 truncation variant of hST6Gal-I
<400> 3
   Glu Lys Lys Lys Gly Ser Tyr Tyr Asp Ser Phe Lys Leu Gln Thr Lys
   1               5                   10                  15
   Glu Phe Gln Val Leu Lys Ser Leu Gly Lys Leu Ala Met Gly Ser Asp
               20                  25                  30
   Ser Gln Ser Val Ser Ser Ser Ser Thr Gln Asp Pro His Arg Gly Arg
           35                  40                  45
   Gln Thr Leu Gly Ser Leu Arg Gly Leu Ala Lys Ala Lys Pro Glu Ala
       50                  55                  60
   Ser Phe Gln Val Trp Asn Lys Asp Ser Ser Ser Lys Asn Leu Ile Pro
   65                  70                  75                  80
   Arg Leu Gln Lys Ile Trp Lys Asn Tyr Leu Ser Met Asn Lys Tyr Lys
                   85                  90                  95
   Val Ser Tyr Lys Gly Pro Gly Pro Gly Ile Lys Phe Ser Ala Glu Ala
               100                 105                 110
   Leu Arg Cys His Leu Arg Asp His Val Asn Val Ser Met Val Glu Val
           115                 120                 125
   Thr Asp Phe Pro Phe Asn Thr Ser Glu Trp Glu Gly Tyr Leu Pro Lys
       130                 135                 140
   Glu Ser Ile Arg Thr Lys Ala Gly Pro Trp Gly Arg Cys Ala Val Val
   145                 150                 155                 160
   Ser Ser Ala Gly Ser Leu Lys Ser Ser Gln Leu Gly Arg Glu Ile Asp
                   165                 170                 175
   Asp His Asp Ala Val Leu Arg Phe Asn Gly Ala Pro Thr Ala Asn Phe
               180                 185                 190
   Gln Gln Asp Val Gly Thr Lys Thr Thr Ile Arg Leu Met Asn Ser Gln
           195                 200                 205
   Leu Val Thr Thr Glu Lys Arg Phe Leu Lys Asp Ser Leu Tyr Asn Glu
       210                 215                 220
   Gly Ile Leu Ile Val Trp Asp Pro Ser Val Tyr His Ser Asp Ile Pro
   225                 230                 235                 240
   Lys Trp Tyr Gln Asn Pro Asp Tyr Asn Phe Phe Asn Asn Tyr Lys Thr
                   245                 250                 255
   Tyr Arg Lys Leu His Pro Asn Gln Pro Phe Tyr Ile Leu Lys Pro Gln
               260                 265                 270
   Met Pro Trp Glu Leu Trp Asp Ile Leu Gln Glu Ile Ser Pro Glu Glu
           275                 280                 285
   Ile Gln Pro Asn Pro Pro Ser Ser Gly Met Leu Gly Ile Ile Ile Met
       290                 295                 300
   Met Thr Leu Cys Asp Gln Val Asp Ile Tyr Glu Phe Leu Pro Ser Lys
   305                 310                 315                 320
   Arg Lys Thr Asp Val Cys Tyr Tyr Tyr Gln Lys Phe Phe Asp Ser Ala
                   325                 330                 335
   Cys Thr Met Gly Ala Tyr His Pro Leu Leu Tyr Glu Lys Asn Leu Val
               340                 345                 350
   Lys His Leu Asn Gln Gly Thr Asp Glu Asp Ile Tyr Leu Leu Gly Lys
           355                 360                 365
   Ala Thr Leu Pro Gly Phe Arg Thr Ile His Cys
       370                 375
<210> 4
   <211> 358
   <212> PRT
   <213> Artificial Sequence
<220>
   <223> delta48 truncation variant of hST6Gal-I
<400> 4
   Lys Ser Leu Gly Lys Leu Ala Met Gly Ser Asp Ser Gln Ser Val Ser
   1               5                   10                  15
   Ser Ser Ser Thr Gln Asp Pro His Arg Gly Arg Gln Thr Leu Gly Ser
               20                  25                  30
   Leu Arg Gly Leu Ala Lys Ala Lys Pro Glu Ala Ser Phe Gln Val Trp
           35                  40                  45
   Asn Lys Asp Ser Ser Ser Lys Asn Leu Ile Pro Arg Leu Gln Lys Ile
       50                  55                  60
   Trp Lys Asn Tyr Leu Ser Met Asn Lys Tyr Lys Val Ser Tyr Lys Gly
   65                  70                  75                  80
   Pro Gly Pro Gly Ile Lys Phe Ser Ala Glu Ala Leu Arg Cys His Leu
                   85                  90                  95
   Arg Asp His Val Asn Val Ser Met Val Glu Val Thr Asp Phe Pro Phe
               100                 105                 110
   Asn Thr Ser Glu Trp Glu Gly Tyr Leu Pro Lys Glu Ser Ile Arg Thr
           115                 120                 125
   Lys Ala Gly Pro Trp Gly Arg Cys Ala Val Val Ser Ser Ala Gly Ser
       130                 135                 140
   Leu Lys Ser Ser Gln Leu Gly Arg Glu Ile Asp Asp His Asp Ala Val
   145                 150                 155                 160
   Leu Arg Phe Asn Gly Ala Pro Thr Ala Asn Phe Gln Gln Asp Val Gly
                   165                 170                 175
   Thr Lys Thr Thr Ile Arg Leu Met Asn Ser Gln Leu Val Thr Thr Glu
               180                 185                 190
   Lys Arg Phe Leu Lys Asp Ser Leu Tyr Asn Glu Gly Ile Leu Ile Val
   195 200 205
   Trp Asp Pro Ser Val Tyr His Ser Asp Ile Pro Lys Trp Tyr Gln Asn
   210 215 220
   Pro Asp Tyr Asn Phe Phe Asn Asn Tyr Lys Thr Tyr Arg Lys Leu His
   225 230 235 240
   Pro Asn Gln Pro Phe Tyr Ile Leu Lys Pro Gln Met Pro Trp Glu Leu
   245 250 255
   Trp Asp Ile Leu Gln Glu Ile Ser Pro Glu Glu Ile Gln Pro Asn Pro
   260 265 270
   Pro Ser Ser Gly Met Leu Gly Ile Ile Ile Met Met Thr Leu Cys Asp
   275 280 285
   Gln Val Asp Ile Tyr Glu Phe Leu Pro Ser Lys Arg Lys Thr Asp Val
   290 295 300
   Cys Tyr Tyr Tyr Gln Lys Phe Phe Asp Ser Ala Cys Thr Met Gly Ala
   305 310 315 320
   Tyr His Pro Leu Leu Tyr Glu Lys Asn Leu Val Lys His Leu Asn Gln
   325 330 335
   Gly Thr Asp Glu Asp Ile Tyr Leu Leu Gly Lys Ala Thr Leu Pro Gly
   340 345 350
   Phe Arg Thr Ile His Cys
   355
<210> 5
   <211> 344
   <212> PRT
   <213> Artificial Sequence
<220>
   <223> delta62 truncation variant of hST6Gal-I
<400> 5
   Val Ser Ser Ser Ser Thr Gln Asp Pro His Arg Gly Arg Gln Thr Leu
   1 5 10 15
   Gly Ser Leu Arg Gly Leu Ala Lys Ala Lys Pro Glu Ala Ser Phe Gln
   20 25 30
   Val Trp Asn Lys Asp Ser Ser Ser Lys Asn Leu Ile Pro Arg Leu Gln
           35                  40                  45
   Lys Ile Trp Lys Asn Tyr Leu Ser Met Asn Lys Tyr Lys Val Ser Tyr
       50                  55                  60
   Lys Gly Pro Gly Pro Gly Ile Lys Phe Ser Ala Glu Ala Leu Arg Cys
   65                  70                  75                  80
   His Leu Arg Asp His Val Asn Val Ser Met Val Glu Val Thr Asp Phe
                   85                  90                  95
   Pro Phe Asn Thr Ser Glu Trp Glu Gly Tyr Leu Pro Lys Glu Ser Ile
               100                 105                 110
   Arg Thr Lys Ala Gly Pro Trp Gly Arg Cys Ala Val Val Ser Ser Ala
           115                 120                 125
   Gly Ser Leu Lys Ser Ser Gln Leu Gly Arg Glu Ile Asp Asp His Asp
       130                 135                 140
   Ala Val Leu Arg Phe Asn Gly Ala Pro Thr Ala Asn Phe Gln Gln Asp
   145                 150                 155                 160
   Val Gly Thr Lys Thr Thr Ile Arg Leu Met Asn Ser Gln Leu Val Thr
                   165                 170                 175
   Thr Glu Lys Arg Phe Leu Lys Asp Ser Leu Tyr Asn Glu Gly Ile Leu
               180                 185                 190
   Ile Val Trp Asp Pro Ser Val Tyr His Ser Asp Ile Pro Lys Trp Tyr
           195                 200                 205
   Gln Asn Pro Asp Tyr Asn Phe Phe Asn Asn Tyr Lys Thr Tyr Arg Lys
       210                 215                 220
   Leu His Pro Asn Gln Pro Phe Tyr Ile Leu Lys Pro Gln Met Pro Trp
   225                 230                 235                 240
   Glu Leu Trp Asp Ile Leu Gln Glu Ile Ser Pro Glu Glu Ile Gln Pro
                   245                 250                 255
   Asn Pro Pro Ser Ser Gly Met Leu Gly Ile Ile Ile Met Met Thr Leu
               260                 265                 270
   Cys Asp Gln Val Asp Ile Tyr Glu Phe Leu Pro Ser Lys Arg Lys Thr
           275                 280                 285
   Asp Val Cys Tyr Tyr Tyr Gln Lys Phe Phe Asp Ser Ala Cys Thr Met
       290                 295                 300
   Gly Ala Tyr His Pro Leu Leu Tyr Glu Lys Asn Leu Val Lys His Leu
   305                 310                 315                 320
   Asn Gln Gly Thr Asp Glu Asp Ile Tyr Leu Leu Gly Lys Ala Thr Leu
                   325                 330                 335
   Pro Gly Phe Arg Thr Ile His Cys
               340
<210> 6
   <211> 317
   <212> PRT
   <213> Artificial Sequence
<220>
   <223> delta89 truncation variant of hST6Gal-I
<400> 6
   Glu Ala Ser Phe Gln Val Trp Asn Lys Asp Ser Ser Ser Lys Asn Leu
   1               5                   10                  15
   Ile Pro Arg Leu Gln Lys Ile Trp Lys Asn Tyr Leu Ser Met Asn Lys
               20                  25                  30
   Tyr Lys Val Ser Tyr Lys Gly Pro Gly Pro Gly Ile Lys Phe Ser Ala
           35                  40                  45
   Glu Ala Leu Arg Cys His Leu Arg Asp His Val Asn Val Ser Met Val
       50                  55                  60
   Glu Val Thr Asp Phe Pro Phe Asn Thr Ser Glu Trp Glu Gly Tyr Leu
   65                  70                  75                  80
   Pro Lys Glu Ser Ile Arg Thr Lys Ala Gly Pro Trp Gly Arg Cys Ala
                   85                  90                  95
   Val Val Ser Ser Ala Gly Ser Leu Lys Ser Ser Gln Leu Gly Arg Glu
               100                 105                 110
   Ile Asp Asp His Asp Ala Val Leu Arg Phe Asn Gly Ala Pro Thr Ala
           115                 120                 125
   Asn Phe Gln Gln Asp Val Gly Thr Lys Thr Thr Ile Arg Leu Met Asn
       130                 135                 140
   Ser Gln Leu Val Thr Thr Glu Lys Arg Phe Leu Lys Asp Ser Leu Tyr
   145                 150                 155                 160
   Asn Glu Gly Ile Leu Ile Val Trp Asp Pro Ser Val Tyr His Ser Asp
                   165                 170                 175
   Ile Pro Lys Trp Tyr Gln Asn Pro Asp Tyr Asn Phe Phe Asn Asn Tyr
               180                 185                 190
   Lys Thr Tyr Arg Lys Leu His Pro Asn Gln Pro Phe Tyr Ile Leu Lys
           195                 200                 205
   Pro Gln Met Pro Trp Glu Leu Trp Asp Ile Leu Gln Glu Ile Ser Pro
       210                 215                 220
   Glu Glu Ile Gln Pro Asn Pro Pro Ser Ser Gly Met Leu Gly Ile Ile
   225                 230                 235                 240
   Ile Met Met Thr Leu Cys Asp Gln Val Asp Ile Tyr Glu Phe Leu Pro
                   245                 250                 255
   Ser Lys Arg Lys Thr Asp Val Cys Tyr Tyr Tyr Gln Lys Phe Phe Asp
               260                 265                 270
   Ser Ala Cys Thr Met Gly Ala Tyr His Pro Leu Leu Tyr Glu Lys Asn
           275                 280                 285
   Leu Val Lys His Leu Asn Gln Gly Thr Asp Glu Asp Ile Tyr Leu Leu
       290                 295                 300
   Gly Lys Ala Thr Leu Pro Gly Phe Arg Thr Ile His Cys
   305                 310                 315
<210> 7
   <211> 298
   <212> PRT
   <213> Artificial Sequence
<220>
   <223> delta108 truncation variant of hST6Gal-I
<400> 7
   Leu Gln Lys Ile Trp Lys Asn Tyr Leu Ser Met Asn Lys Tyr Lys Val
   1               5                   10                  15
   Ser Tyr Lys Gly Pro Gly Pro Gly Ile Lys Phe Ser Ala Glu Ala Leu
               20                  25                  30
   Arg Cys His Leu Arg Asp His Val Asn Val Ser Met Val Glu Val Thr
           35                  40                  45
   Asp Phe Pro Phe Asn Thr Ser Glu Trp Glu Gly Tyr Leu Pro Lys Glu
       50                  55                  60
   Ser Ile Arg Thr Lys Ala Gly Pro Trp Gly Arg Cys Ala Val Val Ser
   65                  70                  75                  80
   Ser Ala Gly Ser Leu Lys Ser Ser Gln Leu Gly Arg Glu Ile Asp Asp
                   85                  90                  95
   His Asp Ala Val Leu Arg Phe Asn Gly Ala Pro Thr Ala Asn Phe Gln
               100                 105                 110
   Gln Asp Val Gly Thr Lys Thr Thr Ile Arg Leu Met Asn Ser Gln Leu
           115                 120                 125
   Val Thr Thr Glu Lys Arg Phe Leu Lys Asp Ser Leu Tyr Asn Glu Gly
       130                 135                 140
   Ile Leu Ile Val Trp Asp Pro Ser Val Tyr His Ser Asp Ile Pro Lys
   145                 150                 155                 160
   Trp Tyr Gln Asn Pro Asp Tyr Asn Phe Phe Asn Asn Tyr Lys Thr Tyr
                   165                 170                 175
   Arg Lys Leu His Pro Asn Gln Pro Phe Tyr Ile Leu Lys Pro Gln Met
               180                 185                 190
   Pro Trp Glu Leu Trp Asp Ile Leu Gln Glu Ile Ser Pro Glu Glu Ile
           195                 200                 205
   Gln Pro Asn Pro Pro Ser Ser Gly Met Leu Gly Ile Ile Ile Met Met
       210                 215                 220
   Thr Leu Cys Asp Gln Val Asp Ile Tyr Glu Phe Leu Pro Ser Lys Arg
   225                 230                 235                 240
   Lys Thr Asp Val Cys Tyr Tyr Tyr Gln Lys Phe Phe Asp Ser Ala Cys
                   245                 250                 255
   Thr Met Gly Ala Tyr His Pro Leu Leu Tyr Glu Lys Asn Leu Val Lys
               260                 265                 270
   His Leu Asn Gln Gly Thr Asp Glu Asp Ile Tyr Leu Leu Gly Lys Ala
           275                 280                 285
   Thr Leu Pro Gly Phe Arg Thr Ile His Cys
       290                 295
<210> 8
   <211> 1170
   <212> DNA
   <213> Artificial Sequence
<220>
   <223> Nucleotide sequence encoding N-terminally truncated delta27 hST6Gal-I for expression in Pichia pastoris
<400> 8
   agaggttctc atcatcatca tcatcatatg gagaaaaaga agggatcata ctatgatagt      60
   ttcaaactgc agaccaaaga gtttcaggtc ttgaaatcct taggcaagtt agctatgggg     120
   tccgattccc aatctgtttc ctcatcctcc actcaagacc cacacagagg tagacaaact     180
   ttgggttcct tgagaggttt ggctaaggct aagccagaag cttctttcca ggtttggaac     240
   aaggactcct catccaagaa cttgatccca agattgcaga agatttggaa gaactacttg     300
   tccatgaaca agtacaaggt ttcctacaag ggtccaggtc caggtattaa gttctctgct     360
   gaggctttga gatgtcactt gagagatcac gttaacgttt ccatggttga ggttactgac     420
   ttcccattca acacttccga gtgggaaggt tacttgccaa aagagtccat cagaactaag     480
   gctggtccat ggggtagatg tgctgttgtt tcttctgctg gttccttgaa gtcctcccag     540
   ttgggtagag aaattgatga ccacgacgct gttttgagat tcaacggtgc tccaactgct     600
   aacttccaac aggacgttgg tactaagact actatcagat tgatgaactc ccagttggtt     660
   actactgaga agagattctt gaaggactcc ttgtacaacg agggtatctt gatcgtttgg     720
   gacccatctg tttaccactc cgacatccca aagtggtatc agaacccaga ctacaacttc     780
   ttcaacaact acaagactta cagaaagttg cacccaaacc agccattcta catcttgaag     840
   ccacagatgc catgggagtt gtgggacatc ttgcaagaaa tctccccaga agagatccag     900
   ccaaaccctc catcttctgg tatgttgggt atcatcatca tgatgacttt gtgtgaccag     960
   gttgacatct acgagttctt gccatccaag agaaagactg acgtttgtta ctactaccag    1020
   aagttcttcg actccgcttg tactatgggt gcttaccacc ctttgttgta cgagaagaac    1080
   ttggttaagc acttgaacca gggtactgac gaggacatct acttgttggg taaggctact    1140
   ttgccaggtt tcagaactat ccactgttga                                    1170
<210> 9
   <211> 389
   <212> PRT
   <213> Artificial Sequence
<220>
   <223> His-tagged delta27 hST6-Gal-I polypeptide
<400> 9
   Arg Gly Ser His His His His His His Met Glu Lys Lys Lys Gly Ser
   1 5 10 15
   Tyr Tyr Asp Ser Phe Lys Leu Gln Thr Lys Glu Phe Gln Val Leu Lys
   20 25 30
   Ser Leu Gly Lys Leu Ala Met Gly Ser Asp Ser Gln Ser Val Ser Ser
   35 40 45
   Ser Ser Thr Gln Asp Pro His Arg Gly Arg Gln Thr Leu Gly Ser Leu
   50 55 60
   Arg Gly Leu Ala Lys Ala Lys Pro Glu Ala Ser Phe Gln Val Trp Asn
   65 70 75 80
   Lys Asp Ser Ser Ser Lys Asn Leu Ile Pro Arg Leu Gln Lys Ile Trp
   85 90 95
   Lys Asn Tyr Leu Ser Met Asn Lys Tyr Lys Val Ser Tyr Lys Gly Pro
   100 105 110
   Gly Pro Gly Ile Lys Phe Ser Ala Glu Ala Leu Arg Cys His Leu Arg
   115 120 125
   Asp His Val Asn Val Ser Met Val Glu Val Thr Asp Phe Pro Phe Asn
   130 135 140
   Thr Ser Glu Trp Glu Gly Tyr Leu Pro Lys Glu Ser Ile Arg Thr Lys
   145 150 155 160
   Ala Gly Pro Trp Gly Arg Cys Ala Val Val Ser Ser Ala Gly Ser Leu
   165 170 175
   Lys Ser Ser Gln Leu Gly Arg Glu Ile Asp Asp His Asp Ala Val Leu
   180 185 190
   Arg Phe Asn Gly Ala Pro Thr Ala Asn Phe Gln Gln Asp Val Gly Thr
   195 200 205
   Lys Thr Thr Ile Arg Leu Met Asn Ser Gln Leu Val Thr Thr Glu Lys
   210 215 220
   Arg Phe Leu Lys Asp Ser Leu Tyr Asn Glu Gly Ile Leu Ile Val Trp
   225 230 235 240
   Asp Pro Ser Val Tyr His Ser Asp Ile Pro Lys Trp Tyr Gln Asn Pro
                   245                 250                 255
   Asp Tyr Asn Phe Phe Asn Asn Tyr Lys Thr Tyr Arg Lys Leu His Pro
               260                 265                 270
   Asn Gln Pro Phe Tyr Ile Leu Lys Pro Gln Met Pro Trp Glu Leu Trp
           275                 280                 285
   Asp Ile Leu Gln Glu Ile Ser Pro Glu Glu Ile Gln Pro Asn Pro Pro
       290                 295                 300
   Ser Ser Gly Met Leu Gly Ile Ile Ile Met Met Thr Leu Cys Asp Gln
   305                 310                 315                 320
   Val Asp Ile Tyr Glu Phe Leu Pro Ser Lys Arg Lys Thr Asp Val Cys
                   325                 330                 335
   Tyr Tyr Tyr Gln Lys Phe Phe Asp Ser Ala Cys Thr Met Gly Ala Tyr
               340                 345                 350
   His Pro Leu Leu Tyr Glu Lys Asn Leu Val Lys His Leu Asn Gln Gly
           355                 360                 365
   Thr Asp Glu Asp Ile Tyr Leu Leu Gly Lys Ala Thr Leu Pro Gly Phe
       370                 375                 380
   Arg Thr Ile His Cys
   385
<210> 10
   <211> 1107
   <212> DNA
   <213> Artificial Sequence
<220>
   <223> Nucleotide sequence encoding N-terminally truncated delta48 hST6Gal-I for expression in Pichia pastoris
<400> 10
   agaggttctc atcatcatca tcatcatatg aaatccttag gcaagttagc tatggggtcc 60
   gattcccaat ctgtttcctc atcctccact caagacccac acagaggtag acaaactttg 120
   ggttccttga gaggtttggc taaggctaag ccagaagctt ctttccaggt ttggaacaag 180
   gactcctcat ccaagaactt gatcccaaga ttgcagaaga tttggaagaa ctacttgtcc 240
   atgaacaagt acaaggtttc ctacaagggt ccaggtccag gtattaagtt ctctgctgag 300
   gctttgagat gtcacttgag agatcacgtt aacgtttcca tggttgaggt tactgacttc 360
   ccattcaaca cttccgagtg ggaaggttac ttgccaaaag agtccatcag aactaaggct 420
   ggtccatggg gtagatgtgc tgttgtttct tctgctggtt ccttgaagtc ctcccagttg 480
   ggtagagaaa ttgatgacca cgacgctgtt ttgagattca acggtgctcc aactgctaac 540
   ttccaacagg acgttggtac taagactact atcagattga tgaactccca gttggttact 600
   actgagaaga gattcttgaa ggactccttg tacaacgagg gtatcttgat cgtttgggac 660
   ccatctgttt accactccga catcccaaag tggtatcaga acccagacta caacttcttc 720
   aacaactaca agacttacag aaagttgcac ccaaaccagc cattctacat cttgaagcca 780
   cagatgccat gggagttgtg ggacatcttg caagaaatct ccccagaaga gatccagcca 840
   aaccctccat cttctggtat gttgggtatc atcatcatga tgactttgtg tgaccaggtt 900
   gacatctacg agttcttgcc atccaagaga aagactgacg tttgttacta ctaccagaag 960
   ttcttcgact ccgcttgtac tatgggtgct taccaccctt tgttgtacga gaagaacttg 1020
   gttaagcact tgaaccaggg tactgacgag gacatctact tgttgggtaa ggctactttg 1080
   ccaggtttca gaactatcca ctgttga 1107
<210> 11
   <211> 368
   <212> PRT
   <213> Artificial Sequence
<220>
   <223> His-tagged delta48 hST6-Gal-I polypeptide
<400> 11
   Arg Gly Ser His His His His His His Met Lys Ser Leu Gly Lys Leu
   1               5                   10                  15
   Ala Met Gly Ser Asp Ser Gln Ser Val Ser Ser Ser Ser Thr Gln Asp
               20                  25                  30
   Pro His Arg Gly Arg Gln Thr Leu Gly Ser Leu Arg Gly Leu Ala Lys
           35                  40                  45
   Ala Lys Pro Glu Ala Ser Phe Gln Val Trp Asn Lys Asp Ser Ser Ser
       50                  55                  60
   Lys Asn Leu Ile Pro Arg Leu Gln Lys Ile Trp Lys Asn Tyr Leu Ser
   65                  70                  75                  80
   Met Asn Lys Tyr Lys Val Ser Tyr Lys Gly Pro Gly Pro Gly Ile Lys
                   85                  90                  95
   Phe Ser Ala Glu Ala Leu Arg Cys His Leu Arg Asp His Val Asn Val
               100                 105                 110
   Ser Met Val Glu Val Thr Asp Phe Pro Phe Asn Thr Ser Glu Trp Glu
           115                 120                 125
   Gly Tyr Leu Pro Lys Glu Ser Ile Arg Thr Lys Ala Gly Pro Trp Gly
       130                 135                 140
   Arg Cys Ala Val Val Ser Ser Ala Gly Ser Leu Lys Ser Ser Gln Leu
   145                 150                 155                 160
   Gly Arg Glu Ile Asp Asp His Asp Ala Val Leu Arg Phe Asn Gly Ala
                   165                 170                 175
   Pro Thr Ala Asn Phe Gln Gln Asp Val Gly Thr Lys Thr Thr Ile Arg
               180                 185                 190
   Leu Met Asn Ser Gln Leu Val Thr Thr Glu Lys Arg Phe Leu Lys Asp
           195                 200                 205
   Ser Leu Tyr Asn Glu Gly Ile Leu Ile Val Trp Asp Pro Ser Val Tyr
       210                 215                 220
   His Ser Asp Ile Pro Lys Trp Tyr Gln Asn Pro Asp Tyr Asn Phe Phe
   225                 230                 235                 240
   Asn Asn Tyr Lys Thr Tyr Arg Lys Leu His Pro Asn Gln Pro Phe Tyr
                   245                 250                 255
   Ile Leu Lys Pro Gln Met Pro Trp Glu Leu Trp Asp Ile Leu Gln Glu
               260                 265                 270
   Ile Ser Pro Glu Glu Ile Gln Pro Asn Pro Pro Ser Ser Gly Met Leu
           275                 280                 285
   Gly Ile Ile Ile Met Met Thr Leu Cys Asp Gln Val Asp Ile Tyr Glu
       290                 295                 300
   Phe Leu Pro Ser Lys Arg Lys Thr Asp Val Cys Tyr Tyr Tyr Gln Lys
   305                 310                 315                 320
   Phe Phe Asp Ser Ala Cys Thr Met Gly Ala Tyr His Pro Leu Leu Tyr
                   325                 330                 335
   Glu Lys Asn Leu Val Lys His Leu Asn Gln Gly Thr Asp Glu Asp Ile
               340                 345                 350
   Tyr Leu Leu Gly Lys Ala Thr Leu Pro Gly Phe Arg Thr Ile His Cys
           355                 360                 365
<210> 12
   <211> 1065
   <212> DNA
   <213> Artificial Sequence
<220>
   <223> Nucleotide sequence encoding N-terminally truncated delta62 hST6Gal-I for expression in Pichia pastoris
<400> 12
   agaggttctc atcatcatca tcatcatatg gtttcctcat cctccactca agacccacac      60
   agaggtagac aaactttggg ttccttgaga ggtttggcta aggctaagcc agaagcttct     120
   ttccaggttt ggaacaagga ctcctcatcc aagaacttga tcccaagatt gcagaagatt     180
   tggaagaact acttgtccat gaacaagtac aaggtttcct acaagggtcc aggtccaggt     240
   attaagttct ctgctgaggc tttgagatgt cacttgagag atcacgttaa cgtttccatg     300
   gttgaggtta ctgacttccc attcaacact tccgagtggg aaggttactt gccaaaagag     360
   tccatcagaa ctaaggctgg tccatggggt agatgtgctg ttgtttcttc tgctggttcc     420
   ttgaagtcct cccagttggg tagagaaatt gatgaccacg acgctgtttt gagattcaac     480
   ggtgctccaa ctgctaactt ccaacaggac gttggtacta agactactat cagattgatg     540
   aactcccagt tggttactac tgagaagaga ttcttgaagg actccttgta caacgagggt     600
   atcttgatcg tttgggaccc atctgtttac cactccgaca tcccaaagtg gtatcagaac     660
   ccagactaca acttcttcaa caactacaag acttacagaa agttgcaccc aaaccagcca     720
   ttctacatct tgaagccaca gatgccatgg gagttgtggg acatcttgca agaaatctcc     780
   ccagaagaga tccagccaaa ccctccatct tctggtatgt tgggtatcat catcatgatg     840
   actttgtgtg accaggttga catctacgag ttcttgccat ccaagagaaa gactgacgtt     900
   tgttactact accagaagtt cttcgactcc gcttgtacta tgggtgctta ccaccctttg      960
   ttgtacgaga agaacttggt taagcacttg aaccagggta ctgacgagga catctacttg    1020
   ttgggtaagg ctactttgcc aggtttcaga actatccact gttga                    1065
<210> 13
   <211> 354
   <212> PRT
   <213> Artificial Sequence
<220>
   <223> His-tagged delta62 hST6-Gal-I polypeptide
<400> 13
   Arg Gly Ser His His His His His His Met Val Ser Ser Ser Ser Thr
   1               5                   10                  15
   Gln Asp Pro His Arg Gly Arg Gln Thr Leu Gly Ser Leu Arg Gly Leu
   20 25 30
   Ala Lys Ala Lys Pro Glu Ala Ser Phe Gln Val Trp Asn Lys Asp Ser
   35 40 45
   Ser Ser Lys Asn Leu Ile Pro Arg Leu Gln Lys Ile Trp Lys Asn Tyr
   50 55 60
   Leu Ser Met Asn Lys Tyr Lys Val Ser Tyr Lys Gly Pro Gly Pro Gly
   65 70 75 80
   Ile Lys Phe Ser Ala Glu Ala Leu Arg Cys His Leu Arg Asp His Val
   85 90 95
   Asn Val Ser Met Val Glu Val Thr Asp Phe Pro Phe Asn Thr Ser Glu
   100 105 110
   Trp Glu Gly Tyr Leu Pro Lys Glu Ser Ile Arg Thr Lys Ala Gly Pro
   115 120 125
   Trp Gly Arg Cys Ala Val Val Ser Ser Ala Gly Ser Leu Lys Ser Ser
   130 135 140
   Gln Leu Gly Arg Glu Ile Asp Asp His Asp Ala Val Leu Arg Phe Asn
   145 150 155 160
   Gly Ala Pro Thr Ala Asn Phe Gln Gln Asp Val Gly Thr Lys Thr Thr
   165 170 175
   Ile Arg Leu Met Asn Ser Gln Leu Val Thr Thr Glu Lys Arg Phe Leu
   180 185 190
   Lys Asp Ser Leu Tyr Asn Glu Gly Ile Leu Ile Val Trp Asp Pro Ser
   195 200 205
   Val Tyr His Ser Asp Ile Pro Lys Trp Tyr Gln Asn Pro Asp Tyr Asn
   210 215 220
   Phe Phe Asn Asn Tyr Lys Thr Tyr Arg Lys Leu His Pro Asn Gln Pro
   225 230 235 240
   Phe Tyr Ile Leu Lys Pro Gln Met Pro Trp Glu Leu Trp Asp Ile Leu
   245 250 255
   Gln Glu Ile Ser Pro Glu Glu Ile Gln Pro Asn Pro Pro Ser Ser Gly
   260 265 270
   Met Leu Gly Ile Ile Ile Met Met Thr Leu Cys Asp Gln Val Asp Ile
           275                 280                 285
   Tyr Glu Phe Leu Pro Ser Lys Arg Lys Thr Asp Val Cys Tyr Tyr Tyr
       290                 295                 300
   Gln Lys Phe Phe Asp Ser Ala Cys Thr Met Gly Ala Tyr His Pro Leu
   305                 310                 315                 320
   Leu Tyr Glu Lys Asn Leu Val Lys His Leu Asn Gln Gly Thr Asp Glu
                   325                 330                 335
   Asp Ile Tyr Leu Leu Gly Lys Ala Thr Leu Pro Gly Phe Arg Thr Ile
               340                 345                 350
   His Cys
<210> 14
   <211> 984
   <212> DNA
   <213> Artificial Sequence
<220>
   <223> Nucleotide sequence encoding N-terminally truncated delta89 hST6Gal-I for expression in Pichia pastoris
<400> 14
   agaggttctc atcatcatca tcatcatatg gaagcttctt tccaggtttg gaacaaggac      60
   tcctcatcca agaacttgat cccaagattg cagaagattt ggaagaacta cttgtccatg     120
   aacaagtaca aggtttccta caagggtcca ggtccaggta ttaagttctc tgctgaggct     180
   ttgagatgtc acttgagaga tcacgttaac gtttccatgg ttgaggttac tgacttccca     240
   ttcaacactt ccgagtggga aggttacttg ccaaaagagt ccatcagaac taaggctggt     300
   ccatggggta gatgtgctgt tgtttcttct gctggttcct tgaagtcctc ccagttgggt     360
   agagaaattg atgaccacga cgctgttttg agattcaacg gtgctccaac tgctaacttc     420
   caacaggacg ttggtactaa gactactatc agattgatga actcccagtt ggttactact     480
   gagaagagat tcttgaagga ctccttgtac aacgagggta tcttgatcgt ttgggaccca     540
   tctgtttacc actccgacat cccaaagtgg tatcagaacc cagactacaa cttcttcaac     600
   aactacaaga cttacagaaa gttgcaccca aaccagccat tctacatctt gaagccacag     660
   atgccatggg agttgtggga catcttgcaa gaaatctccc cagaagagat ccagccaaac     720
   cctccatctt ctggtatgtt gggtatcatc atcatgatga ctttgtgtga ccaggttgac     780
   atctacgagt tcttgccatc caagagaaag actgacgttt gttactacta ccagaagttc     840
   ttcgactccg cttgtactat gggtgcttac caccctttgt tgtacgagaa gaacttggtt     900
   aagcacttga accagggtac tgacgaggac atctacttgt tgggtaaggc tactttgcca     960
   ggtttcagaa ctatccactg ttga                                            984
<210> 15
   <211> 327
   <212> PRT
   <213> Artificial Sequence
<220>
   <223> His-tagged delta89 hST6-Gal-I polypeptide
<400> 15
   Arg Gly Ser His His His His His His Met Glu Ala Ser Phe Gln Val
   1               5                   10                  15
   Trp Asn Lys Asp Ser Ser Ser Lys Asn Leu Ile Pro Arg Leu Gln Lys
               20                  25                  30
   Ile Trp Lys Asn Tyr Leu Ser Met Asn Lys Tyr Lys Val Ser Tyr Lys
           35                  40                  45
   Gly Pro Gly Pro Gly Ile Lys Phe Ser Ala Glu Ala Leu Arg Cys His
       50                  55                  60
   Leu Arg Asp His Val Asn Val Ser Met Val Glu Val Thr Asp Phe Pro
   65                  70                  75                  80
   Phe Asn Thr Ser Glu Trp Glu Gly Tyr Leu Pro Lys Glu Ser Ile Arg
                   85                  90                  95
   Thr Lys Ala Gly Pro Trp Gly Arg Cys Ala Val Val Ser Ser Ala Gly
               100                 105                 110
   Ser Leu Lys Ser Ser Gln Leu Gly Arg Glu Ile Asp Asp His Asp Ala
           115                 120                 125
   Val Leu Arg Phe Asn Gly Ala Pro Thr Ala Asn Phe Gln Gln Asp Val
       130                 135                 140
   Gly Thr Lys Thr Thr Ile Arg Leu Met Asn Ser Gln Leu Val Thr Thr
   145                 150                 155                 160
   Glu Lys Arg Phe Leu Lys Asp Ser Leu Tyr Asn Glu Gly Ile Leu Ile
                   165                 170                 175
   Val Trp Asp Pro Ser Val Tyr His Ser Asp Ile Pro Lys Trp Tyr Gln
               180                 185                 190
   Asn Pro Asp Tyr Asn Phe Phe Asn Asn Tyr Lys Thr Tyr Arg Lys Leu
           195                 200                 205
   His Pro Asn Gln Pro Phe Tyr Ile Leu Lys Pro Gln Met Pro Trp Glu
       210                 215                 220
   Leu Trp Asp Ile Leu Gln Glu Ile Ser Pro Glu Glu Ile Gln Pro Asn
   225                 230                 235                 240
   Pro Pro Ser Ser Gly Met Leu Gly Ile Ile Ile Met Met Thr Leu Cys
                   245                 250                 255
   Asp Gln Val Asp Ile Tyr Glu Phe Leu Pro Ser Lys Arg Lys Thr Asp
               260                 265                 270
   Val Cys Tyr Tyr Tyr Gln Lys Phe Phe Asp Ser Ala Cys Thr Met Gly
           275                 280                 285
   Ala Tyr His Pro Leu Leu Tyr Glu Lys Asn Leu Val Lys His Leu Asn
       290                 295                 300
   Gln Gly Thr Asp Glu Asp Ile Tyr Leu Leu Gly Lys Ala Thr Leu Pro
   305                 310                 315                 320
   Gly Phe Arg Thr Ile His Cys
                   325
<210> 16
   <211> 927
   <212> DNA
   <213> Artificial Sequence
<220>
   <223> Nucleotide sequence encoding N-terminally truncated delta108 hST6Gal-I for expression in Pichia pastoris
<400> 16
   agaggttctc atcatcatca tcatcatatg ttgcagaaga tttggaagaa ctacttgtcc      60
   atgaacaagt acaaggtttc ctacaagggt ccaggtccag gtattaagtt ctctgctgag     120
   gctttgagat gtcacttgag agatcacgtt aacgtttcca tggttgaggt tactgacttc     180
   ccattcaaca cttccgagtg ggaaggttac ttgccaaaag agtccatcag aactaaggct     240
   ggtccatggg gtagatgtgc tgttgtttct tctgctggtt ccttgaagtc ctcccagttg     300
   ggtagagaaa ttgatgacca cgacgctgtt ttgagattca acggtgctcc aactgctaac     360
   ttccaacagg acgttggtac taagactact atcagattga tgaactccca gttggttact     420
   actgagaaga gattcttgaa ggactccttg tacaacgagg gtatcttgat cgtttgggac     480
   ccatctgttt accactccga catcccaaag tggtatcaga acccagacta caacttcttc      540
   aacaactaca agacttacag aaagttgcac ccaaaccagc cattctacat cttgaagcca      600
   cagatgccat gggagttgtg ggacatcttg caagaaatct ccccagaaga gatccagcca      660
   aaccctccat cttctggtat gttgggtatc atcatcatga tgactttgtg tgaccaggtt      720
   gacatctacg agttcttgcc atccaagaga aagactgacg tttgttacta ctaccagaag      780
   ttcttcgact ccgcttgtac tatgggtgct taccaccctt tgttgtacga gaagaacttg      840
   gttaagcact tgaaccaggg tactgacgag gacatctact tgttgggtaa ggctactttg      900
   ccaggtttca gaactatcca ctgttga                                          927
<210> 17
   <211> 308
   <212> PRT
   <213> Artificial Sequence
<220>
   <223> His-tagged delta108 hST6-Gal-I polypeptide
<400> 17
   Arg Gly Ser His His His His His His Met Leu Gln Lys Ile Trp Lys
   1               5                   10                  15
   Asn Tyr Leu Ser Met Asn Lys Tyr Lys Val Ser Tyr Lys Gly Pro Gly
               20                  25                  30
   Pro Gly Ile Lys Phe Ser Ala Glu Ala Leu Arg Cys His Leu Arg Asp
           35                  40                  45
   His Val Asn Val Ser Met Val Glu Val Thr Asp Phe Pro Phe Asn Thr
       50                  55                  60
   Ser Glu Trp Glu Gly Tyr Leu Pro Lys Glu Ser Ile Arg Thr Lys Ala
   65                  70                  75                  80
   Gly Pro Trp Gly Arg Cys Ala Val Val Ser Ser Ala Gly Ser Leu Lys
                   85                  90                  95
   Ser Ser Gln Leu Gly Arg Glu Ile Asp Asp His Asp Ala Val Leu Arg
               100                 105                 110
   Phe Asn Gly Ala Pro Thr Ala Asn Phe Gln Gln Asp Val Gly Thr Lys
           115                 120                 125
   Thr Thr Ile Arg Leu Met Asn Ser Gln Leu Val Thr Thr Glu Lys Arg
       130                 135                 140
   Phe Leu Lys Asp Ser Leu Tyr Asn Glu Gly Ile Leu Ile Val Trp Asp
   145 150 155 160
   Pro Ser Val Tyr His Ser Asp Ile Pro Lys Trp Tyr Gln Asn Pro Asp
   165 170 175
   Tyr Asn Phe Phe Asn Asn Tyr Lys Thr Tyr Arg Lys Leu His Pro Asn
   180 185 190
   Gln Pro Phe Tyr Ile Leu Lys Pro Gln Met Pro Trp Glu Leu Trp Asp
   195 200 205
   Ile Leu Gln Glu Ile Ser Pro Glu Glu Ile Gln Pro Asn Pro Pro Ser
   210 215 220
   Ser Gly Met Leu Gly Ile Ile Ile Met Met Thr Leu Cys Asp Gln Val
   225 230 235 240
   Asp Ile Tyr Glu Phe Leu Pro Ser Lys Arg Lys Thr Asp Val Cys Tyr
   245 250 255
   Tyr Tyr Gln Lys Phe Phe Asp Ser Ala Cys Thr Met Gly Ala Tyr His
   260 265 270
   Pro Leu Leu Tyr Glu Lys Asn Leu Val Lys His Leu Asn Gln Gly Thr
   275 280 285
   Asp Glu Asp Ile Tyr Leu Leu Gly Lys Ala Thr Leu Pro Gly Phe Arg
   290 295 300
   Thr Ile His Cys
   305
<210> 18
   <211> 1003
   <212> DNA
   <213> Artificial Sequence
<220>
   <223> Expression construct Epo-APPR-delta108 ST6 (109-406) for expression in HEK cells
<220>
   <221> CDS
   <222> (8)..(997)
   <223> open reading frame
<400> 18
   gtcgacc atg ggc gtg cac gaa tgt cct gcc tgg ctg tgg ctg ctg ctg        49
           Met Gly Val His Glu Cys Pro Ala Trp Leu Trp Leu Leu Leu
           1               5                   10
   agc ctg ctg tct ctg cct ctg gga ctg cct gtg ctg ggc gcc cct cct      97
   Ser Leu Leu Ser Leu Pro Leu Gly Leu Pro Val Leu Gly Ala Pro Pro
   15                  20                  25                  30
   aga ctg cag aag atc tgg aag aac tac ctg agc atg aac aag tac aag     145
   Arg Leu Gln Lys Ile Trp Lys Asn Tyr Leu Ser Met Asn Lys Tyr Lys
                   35                  40                  45
   gtg tcc tac aag ggc cct ggc cct ggc atc aag ttt agc gcc gag gcc     193
   Val Ser Tyr Lys Gly Pro Gly Pro Gly Ile Lys Phe Ser Ala Glu Ala
               50                  55                  60
   ctg aga tgc cac ctg agg gat cac gtg aac gtg tcc atg gtg gaa gtg     241
   Leu Arg Cys His Leu Arg Asp His Val Asn Val Ser Met Val Glu Val
           65                  70                  75
   acc gac ttc cca ttc aac acc agc gag tgg gag ggc tac ctg ccc aaa     289
   Thr Asp Phe Pro Phe Asn Thr Ser Glu Trp Glu Gly Tyr Leu Pro Lys
       80                  85                  90
   gag agc atc cgg acc aaa gcc ggc cct tgg gga aga tgt gcc gtg gtg     337
   Glu Ser Ile Arg Thr Lys Ala Gly Pro Trp Gly Arg Cys Ala Val Val
   95                  100                 105                 110
   tct agc gcc ggc agc ctg aag agt agc cag ctg ggc aga gag atc gac     385
   Ser Ser Ala Gly Ser Leu Lys Ser Ser Gln Leu Gly Arg Glu Ile Asp
                   115                 120                 125
   gac cac gac gcc gtg ctg cgg ttc aat ggc gct ccc acc gcc aac ttc     433
   Asp His Asp Ala Val Leu Arg Phe Asn Gly Ala Pro Thr Ala Asn Phe
               130                 135                 140
   cag cag gac gtg ggc acc aag acc acc atc cgg ctg atg aac tcc cag     481
   Gln Gln Asp Val Gly Thr Lys Thr Thr Ile Arg Leu Met Asn Ser Gln
           145                 150                 155
   ctc gtg aca acc gag aag cgg ttc ctg aag gac agc ctg tac aac gag     529
   Leu Val Thr Thr Glu Lys Arg Phe Leu Lys Asp Ser Leu Tyr Asn Glu
       160                 165                 170
   ggc atc ctg atc gtg tgg gac ccc agc gtg tac cac agc gac atc ccc     577
   Gly Ile Leu Ile Val Trp Asp Pro Ser Val Tyr His Ser Asp Ile Pro
   175                 180                 185                 190
   aag tgg tat cag aac ccc gac tac aac ttc ttc aac aac tac aag acc     625
   Lys Trp Tyr Gln Asn Pro Asp Tyr Asn Phe Phe Asn Asn Tyr Lys Thr
                   195                 200                 205
   tac cgg aag ctg cac ccc aac cag ccc ttc tac atc ctg aag ccc cag     673
   Tyr Arg Lys Leu His Pro Asn Gln Pro Phe Tyr Ile Leu Lys Pro Gln
               210                 215                 220
   atg ccc tgg gag ctg tgg gac att ctg cag gaa atc agc ccc gaa gag     721
   Met Pro Trp Glu Leu Trp Asp Ile Leu Gln Glu Ile Ser Pro Glu Glu
           225                 230                 235
   atc cag ccc aac ccc cct agc tct ggc atg ctg ggc atc att atc atg     769
   Ile Gln Pro Asn Pro Pro Ser Ser Gly Met Leu Gly Ile Ile Ile Met
       240                 245                 250
   atg acc ctg tgc gac cag gtg gac atc tac gag ttt ctg ccc tcc aag     817
   Met Thr Leu Cys Asp Gln Val Asp Ile Tyr Glu Phe Leu Pro Ser Lys
   255                 260                 265                 270
   aga aag acc gac gtg tgc tac tac tac cag aag ttc ttc gac agc gcc          865
   Arg Lys Thr Asp Val Cys Tyr Tyr Tyr Gln Lys Phe Phe Asp Ser Ala
                   275                 280                 285
   tgc acc atg gga gcc tac cac cct ctg ctg tac gag aag aac ctc gtg          913
   Cys Thr Met Gly Ala Tyr His Pro Leu Leu Tyr Glu Lys Asn Leu Val
               290                 295                 300
   aag cac ctg aac cag ggc acc gac gag gat atc tac ctg ctg ggc aag          961
   Lys His Leu Asn Gln Gly Thr Asp Glu Asp Ile Tyr Leu Leu Gly Lys
           305                 310                 315
   gcc acc ctg ccc ggc ttc aga acc atc cac tgc tga ggatcc                  1003
   Ala Thr Leu Pro Gly Phe Arg Thr Ile His Cys
       320                 325
<210> 19
   <211> 329
   <212> PRT
   <213> Artificial Sequence
<220>
   <223> Synthetic Construct
<400> 19
   Met Gly Val His Glu Cys Pro Ala Trp Leu Trp Leu Leu Leu Ser Leu
   1               5                   10                  15
   Leu Ser Leu Pro Leu Gly Leu Pro Val Leu Gly Ala Pro Pro Arg Leu
               20                  25                  30
   Gln Lys Ile Trp Lys Asn Tyr Leu Ser Met Asn Lys Tyr Lys Val Ser
           35                  40                  45
   Tyr Lys Gly Pro Gly Pro Gly Ile Lys Phe Ser Ala Glu Ala Leu Arg
       50                  55                  60
   Cys His Leu Arg Asp His Val Asn Val Ser Met Val Glu Val Thr Asp
   65                  70                  75                  80
   Phe Pro Phe Asn Thr Ser Glu Trp Glu Gly Tyr Leu Pro Lys Glu Ser
                   85                  90                  95
   Ile Arg Thr Lys Ala Gly Pro Trp Gly Arg Cys Ala Val Val Ser Ser
               100                 105                 110
   Ala Gly Ser Leu Lys Ser Ser Gln Leu Gly Arg Glu Ile Asp Asp His
           115                 120                 125
   Asp Ala Val Leu Arg Phe Asn Gly Ala Pro Thr Ala Asn Phe Gln Gln
       130                 135                 140
   Asp Val Gly Thr Lys Thr Thr Ile Arg Leu Met Asn Ser Gln Leu Val
   145                 150                 155                 160
   Thr Thr Glu Lys Arg Phe Leu Lys Asp Ser Leu Tyr Asn Glu Gly Ile
                   165                 170                 175
   Leu Ile Val Trp Asp Pro Ser Val Tyr His Ser Asp Ile Pro Lys Trp
               180                 185                 190
   Tyr Gln Asn Pro Asp Tyr Asn Phe Phe Asn Asn Tyr Lys Thr Tyr Arg
           195                 200                 205
   Lys Leu His Pro Asn Gln Pro Phe Tyr Ile Leu Lys Pro Gln Met Pro
       210                 215                 220
   Trp Glu Leu Trp Asp Ile Leu Gln Glu Ile Ser Pro Glu Glu Ile Gln
   225                 230                 235                 240
   Pro Asn Pro Pro Ser Ser Gly Met Leu Gly Ile Ile Ile Met Met Thr
                   245                 250                 255
   Leu Cys Asp Gln Val Asp Ile Tyr Glu Phe Leu Pro Ser Lys Arg Lys
               260                 265                 270
   Thr Asp Val Cys Tyr Tyr Tyr Gln Lys Phe Phe Asp Ser Ala Cys Thr
           275                 280                 285
   Met Gly Ala Tyr His Pro Leu Leu Tyr Glu Lys Asn Leu Val Lys His
       290                 295                 300
   Leu Asn Gln Gly Thr Asp Glu Asp Ile Tyr Leu Leu Gly Lys Ala Thr
   305                 310                 315                 320
   Leu Pro Gly Phe Arg Thr Ile His Cys
                   325

## Description

The present disclosure is directed to glycosyltransferase variants having N-terminal truncation deletions. Contrary to previous findings certain truncations comprising the conserved amino acid motif ("QVWxKDS", SEQ ID NO:2) were found to be compatible with glycosyltransferase enzymatic activity, particularly in a human sialyltransferase (hST6Gal-I). Thus, disclosed are variants of mammalian glycosyltransferase, nucleic acids encoding the same, methods and means for recombinantly producing the variants of mammalian glycosyltransferase and use thereof, particularly for sialylating terminal acceptor groups of glycan moieties being part of glycoproteins such as immunoglobulins.

### Background

Transferases (EC 2) catalyze transfer of a functional group from one substance to another. Glycosyltransferases, a superfamily of enzymes, are involved in synthesizing the carbohydrate portions of glycoproteins, glycolipids and glycosaminoglycans. Specific glycosyltransferases synthesize oligosaccharides by the sequential transfer of the monosaccharide moiety of an activated sugar donor to an acceptor molecule. Hence, a "glycosyltransferase" catalyzes the transfer of a sugar moiety from its nucleotide donor to an acceptor moiety of a polypeptide, lipid, glycoprotein or glycolipid. This process is also known as "glycosylation". A carbohydrate portion which is structural part of e.g. a glycoprotein is also refered to as "glycan". Glycans constitute the most prevalent of all known post-translational protein modifications. Glycans are involved in a wide array of biological recognition processes as diverse as adhesion, immune response, neural cell migration and axonal extension. As structural part of glycoproteins glycans also have a role in protein folding and the support of protein stability and biological activity.

In glycosyltransferase catalysis, the monosaccharide units glucose (Glc), galactose (Gal), N-acetylglucosamine (GlcNAc), N-acetylgalactosamine (GalNAc), glucuronic acid (GlcUA), galacturonic acid (GalUA) and xylose are activated as uridine diphosphate (UDP)-α-D derivatives; arabinose is activated as a UDP-β-L derivative; mannose (Man) and fucose are activated as GDP-α-D and GDP-β-L derivatives, respectively; and sialic acid (= Neu5Ac; = SA) is activated as a CMP derivative of β-D-Neu5Ac.

Many different glycosyltransferases contribute to the synthesis of glycans. The structural diversity of carbohydrate portions of glycoproteins is particularly large and is determined by complex biosynthetic pathways. In eukaryotes the biosynthesis of the glycan-part of glycoproteins takes place in the lumen of the endoplasmatic reticulum ("ER") and the Golgi apparatus. A single (branched or linear) carbohydrate chain of a glycoprotein is typically a N- or an O-linked glycan. During post-translational processing, carbohydrates are typically connected to the polypeptide via asparagine ("N-linked glycosylation"), or via serine or threonine ("O-linked glycosylation"). Synthesis of a glycan, no matter whether N- or O-linked (= "N-/O-linked") is effected by the activity of several different membrane-anchored glycosyltransferases. A glycoprotein may comprise one or more glycan-connected amino acids (= "glycosylation sites"). A specific glycan structure may be linear or branched. Branching is a notable feature of carbohydrates which is in contrast to the linear nature typical for DNA, RNA, and polypeptides. Combined with the large heterogeneity of their basic building blocks, the monosaccharides, glycan structures exhibit high diversity. Furthermore, in members of a particular glycoprotein species the structure of a glycan attached to a particular glycosylation site may vary, thus resulting in microheterogeneity of the respective glycoprotein species, i.e. in a species sharing the same amino acid sequence of the poypeptide portion.

A sialyltransferase (= "ST") is a glycosyltransferase that catalyzes transfer of a sialic acid (= 5-N-acetylneuramic acid = Neu5Ac = NANA) residue from a donor compound to (i) a terminal monosaccharide acceptor group of a glycolipid or a ganglioside, or (ii) to a terminal monosaccharide acceptor group of an N-/O-linked glycan of a glycoprotein. For mammalian sialyltransferases including human ST species there is a common donor compound which is cytidine-5'-monophospho-N-acetylneuraminic acid (= CMP-Neu5Ac = CMP-NANA). Transfer of a sialic acid residue is also referred to as "sialylating" and "sialylation".

In the glycan structure of a sialylated glycoprotein the (one or more) sialyl moiety (moieties) is (are) usually found in terminal position of the oligosaccharide. Owing to the terminal, i.e. exposed position, sialic acid can participate in many different biological recognition phenomena and serve in different kinds of biological interactions. In a glycoprotein more than one sialylation site may be present, i.e. a site capable of serving as a substrate for a sialyltransferase and being an acceptor group suitable for the transfer of a sialic acid residue. Such more than one site can in principle be the termini of a plurality of linear glycan portions anchored at different glycosylation sites of the glycoprotein. Additionally, a branched glycan may have a plurality of sites where sialylation can occur.

According to current knowledge, a terminal sialic acid residue can be found (i) α2 →3 (α2,3) linked to galactosyl-R, (ii) α2→6 (α2,6) linked to galactosyl-R, (iii) α2 →6 (α2,6) linked to N-acetylgalactosaminidyl-R, (iv) α2→6 (α2,6) linked to N-acetylglucosaminidyl-R, and (v) α2→8/9 (α2,8/9) linked to sialidyl-R, wherein -R denotes the rest of the acceptor substrate moiety. Hence, a sialyltransferase active in the biosynthesis of sialylconjugates (= "sialylation") is generally named and classified according to its respective monosaccharide acceptor substrate and according to the 3, 6 or 8/9 position of the glycosidic bond it catalyzes. Accordingly, in the literature known to the art, e.g. in Patel RY, et al, Glycobiology 16 (2006) 108-116, reference to eukaryotic sialyltransferases is made such as (i) ST3Gal, (ii) ST6Gal, (iii) ST6GalNAc, or (v) ST8Sia, depending on the hydroxyl position of the acceptor sugar residue to which the Neu5Ac residue is transferred while forming a glycosidic bond. Reference to sialyltransferases in a more generic way can also be made e.g. as ST3, ST6, ST8; thus, "ST6" specifically encompasses the sialyltransferases catalyzing an α2,6 sialylation.

The disaccharide moiety β-D-galactosyl-1,4-N-acetyl-β-D-glucosamine (= Galβ1,4GlcNAc) is a frequent terminal residue of the antennae of N-linked glycans of glycoproteins, but may be also present in O-linked glycans and in glycolipids. The enzyme β-galactoside-α2,6-sialyltransferase (= "ST6Gal") is able to catalyze α2,6-sialylation of a terminal Galβ1,4GlcNAc of a glycan or a branch of a glycan (= "antenna"). For general aspects thereof, reference is made to the document of DallOlio F. Glycoconjugate Journal 17 (2000) 669-676. In human and in other mammals there appear to be several species of ST6Gal. The present disclosure particularly deals with human β-galactoside-α-2,6-sialyltransferase I (= hST6Gal-I; EC 2.4.99.1 according to IUBMB Enzyme Nomenclature), but is not limited thereto.

The ST6 group of sialyltransferases comprises 2 subgroups, ST6Gal and ST6GalNAc. The activity of ST6Gal enzymes catalyzes transfer of a Neu5Ac residue to the C6 hydroxyl group of a free galactosyl residue being part of terminal Galβ1,4GlcNAc in a glycan or an antenna of a glycan, thereby forming in the glycan a terminal sialic acid residue α2→6 linked to the galactosyl residue of the Galβ1,4GlcNAc moiety. The resulting newly formed terminal moiety in the glycan is Neu5Acα2,6Galβ1,4GlcNAc.

The wild-type polypeptide of human β-galactoside-α-2,6-sialyltransferase I (hST6Gal-I) at the time of filing of the present document was disclosed as "UniProtKB/Swiss-Prot: P15907.1" in the publically accessible NCBI database (http://www.ncbi.nlm.nih.gov/protein/115445). Further information including coding sequences are provided as hyperlinks compiled within the database entry "Gene ID: 6480" (http://www.ncbi.nlm.nih.gov/gene/6480).

Mammalian sialyltransferases share with other mammalian Golgi-resident glycosyltransferases a so-called "type II architecture" with (i) a short cytoplasmic N-terminal tail, (ii) a transmembrane fragment followed by (iii) a stem region of variable length and (iv) a C-terminal catalytic domain facing the lumen of the Golgi apparatus (Donadio S. et al. in Biochimie 85 (2003) 311-321). Mammalian sialyltransferases appear to display significant sequence homology in their catalytic domain. However, even among a large number of eukaryotic glycosyltransferases in general (i.e. a group of enzymes including the sialyltransferases and other glycosyltransferases), a conserved motif on the amino acid sequence level is observed, namely the QVWxKDS consensus motif of SEQ ID NO:2. Human ST6Gal-I ("hST6Gal-I") shown as SEQ ID NO:1 (wild-type sequence) includes this motif, too, notably on the positions 94-100 of the amino acid sequence of the hST6Gal-I wild-type polypeptide as e.g. at the time of filing of the present document disclosed as "UniProtKB/Swiss-Prot: P15907.1" in the publically accessible NCBI database (http://www.ncbi.nlm.nih.gov/protein/115445).

According to the publication of Donadio S. et al. (*supra*), the amino acid sequence of the QVWxKDS consensus motif is essential for the catalytic domain of hST6Gal-I to acquire a biologically active conformation. Donadio S. et al. expressed several N-terminally truncated variants of hST6Gal-I in CHO cells and found that N-terminal deletions comprising the first 35, 48, 60 and 89 amino acids yielded mutant enzymes which nevertheless were still active in transferring sialic acid to exogenous acceptors. But a hST6Gal-I mutant with a N-terminal deletion of the first 100 amino acids was found to be inactive in this respect. Notably, this "Δ100" deletion mutant of hST6Gal-I lacked the highly conserved QVWxKDS motif. Hence, presence of the motif was concluded by Donadio S. et al. (*supra*) to be crucial for promoting sialyltransferase activity.

Surprisingly and contradicting the findings and teachings of Donadio S. et al (*supra*) the authors of the present disclosure found that deletion of a sequence portion comprising the entire conserved QVWxKDS motif in the amino acid sequence of a glycosyltransferase polypeptide can indeed be compatible with glycosyltransferase activity, particularly sialyltransferase activity.

### Summary

The present invention relates to a variant mammalian glycosyltransferase, wherein the polypeptide of the variant comprises an N-terminally truncated amino acid sequence of the wild-type mammalian glycosyltransferase, and wherein the polypeptide of the variant consists of the amino acid sequence from position 109 to position 406 of SEQ ID NO:1. Further, the present invention relates to a nucleotide sequence encoding the polypeptide of a variant mammalian glycosyltransferase according to the invention. Additionally, the present invention relates to an expression vector comprising a target gene operably linked to sequences facilitating expression of the target gene in a host organism transformed with the expression vector, wherein the target gene comprises a nucleotide sequence according to the invention. In addition, the present invention relates to a transformed host organism, wherein the host organism is transformed with an expression vector according to the invention. Further, the present invention relates to a method to recombinantly produce a variant mammalian glycosyltransferase, the method comprising the step of expressing in a transformed host organism a nucleotide sequence encoding the variant mammalian glycosyltransferase according to the invention, wherein a polypeptide is formed, thereby producing the variant mammalian glycosyltransferase. Additionally, the present invention relates to a variant mammalian glycosyltransferase, wherein the polypeptide of the variant consists of the amino acid sequence from position 109 to position 406 of SEQ ID NO:1, obtained by a method according to the invention. In addition, the present invention relates to the use of a variant mammalian glycosyltransferase according to the invention, or a variant of human human β-galactoside-α-2,6-sialyltransferase I obtained by a method according to the invention for transferring a 5-N-acetylneuraminic acid residue from the donor compound cytidine-5'-monophospho-N-acetylneuraminic acid, or from a functional equivalent thereof, to an acceptor, the acceptor being terminal β-D-galactosyl-1,4-N-acetyl-β-D-glucosamine in a glycan moiety of a monoclonal antibody.

In a first aspect there is reported a variant mammalian glycosyltransferase, wherein the polypeptide of the variant comprises an N-terminally truncated amino acid sequence of the wild-type mammalian glycosyltransferase, the truncation comprising the amino acid sequence motif of SEQ ID NO:2, and wherein the variant exhibits glycosyltransferase activity. In a second aspect there is reported a nucleotide sequence encoding the polypeptide of a variant mammalian glycosyltransferase as disclosed in here. In a third aspect there is reported an expression vector comprising a target gene operably linked to sequences facilitating expression of the target gene in a host organism transformed with the expression vector, wherein the target gene comprises a nucleotide sequence as disclosed in here. In a fourth aspect there is reported a transformed host organism, wherein the host organism is transformed with an expression vector as disclosed in here. In a fifth aspect there is reported a method to recombinantly produce a variant mammalian glycosyltransferase, the method comprising the step of expressing in a transformed host organism a nucleotide sequence encoding the variant mammalian glycosyltransferase as disclosed in here, wherein a polypeptide is formed, thereby producing the variant mammalian glycosyltransferase. In a sixth aspect there is reported a glycosyltransferase obtained by a method as disclosed in here. In a seventh aspect there is reported the use of a variant mammalian glycosyltransferase as disclosed in here for transferring a 5-N-acetylneuraminic acid residue from the donor compound cytidine-5'-monophospho-N-acetylneuraminic acid, or from a functional equivalent thereof, to an acceptor, the acceptor being terminal β-D-galactosyl-1,4-N-acetyl-β-D-glucosamine in a glycan moiety of a monoclonal antibody.

### Description of the Figures

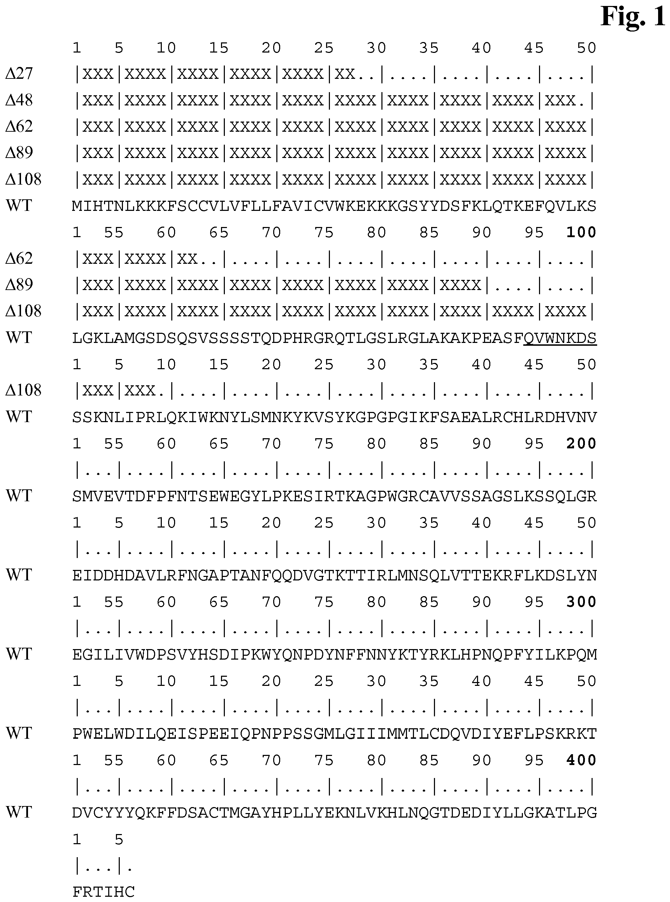

- **Figure 1**: Representation of the amino acid sequence of the wild-type hST6Gal-I polypeptide, and the N-terminal portions thereof which are truncated in the Δ27, Δ48, Δ62, Δ89, and Δ108, variants. The deleted positions in the truncations are symbolized by "X".

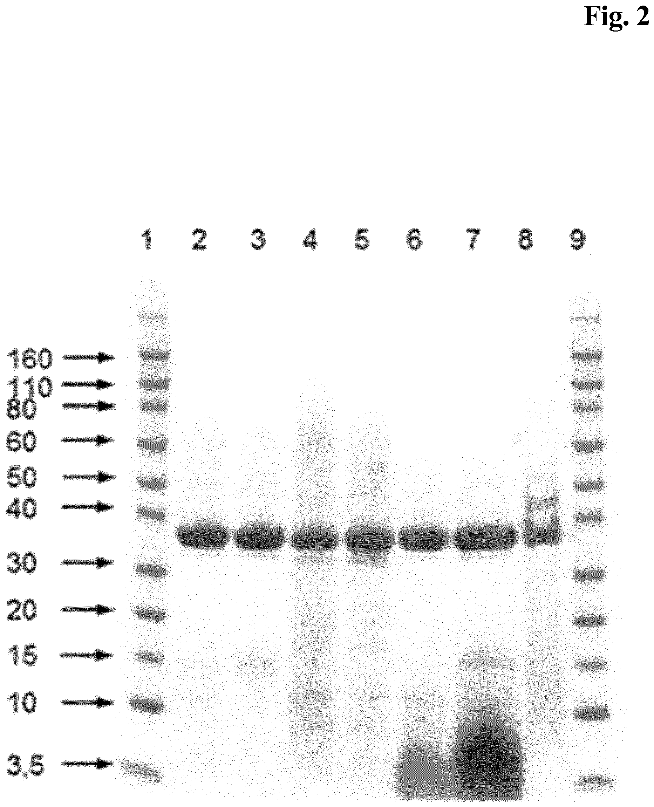

- **Figure 2**: SDS gel after electrophoresis and staining of hST6Gal-I variants expressed in and secreted from *Pichia pastoris.* Lanes 1 and 9 contain a size-standard, molecular weights in kDa according to the standard are indicated to the left. Lane 2: Δ62; Lane 3: Δ48; Lane 4: Δ27 ("clone 103"); Lane 5: Δ27 ("clone 154"); Lane 6: Δ62 ("clone 356"); Lane 7: Δ48 ("clone 9"); Lane 8: Δ89 ("clone 187").

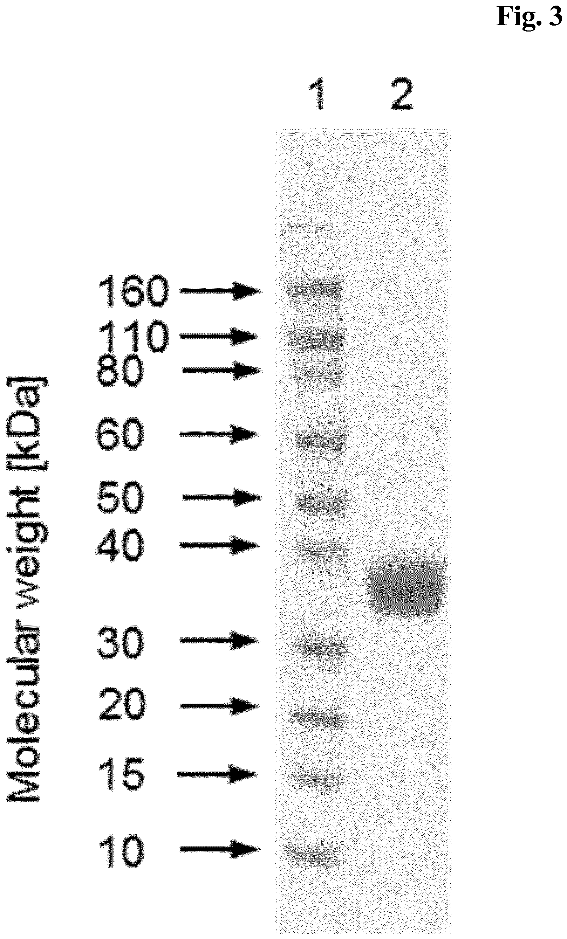

- **Figure 3**: SDS gel after electrophoresis and staining of the Δ108 hST6Gal-I variant transiently expressed in and secreted from HEK cells. Lane 1 contains a size-standard, molecular weights in kDa according to the standard are indicated to the left. Lane 2: Δ108 hST6Gal-I truncation variant (5 µg were loaded on the gel).

### Detailed Description

The terms "a", "an" and "the" generally include plural referents, unless the context clearly indicates otherwise. As used herein, "plurality" is understood to mean more than one. For example, a plurality refers to at least two, three, four, five, or more. Unless specifically stated or obvious from context, as used herein, the term "or" is understood to be inclusive.

Unless specifically stated or obvious from context, as used herein, the term "about" is understood as within a range of normal tolerance in the art, for example within 2 standard deviations of the mean. About can be understood as within 10%, 9%, 8%, 7%, 6%, 5%, 4%, 3%, 2%, 1%, 0.5%, 0.1%, 0.05%, or 0.01% of the stated value. Unless otherwise clear from context, all numerical values provided herein can be modified by the term about.

The term "amino acid" generally refers to any monomer unit that can be incorporated into a peptide, polypeptide, or protein. As used herein, the term "amino acid" includes the following twenty natural or genetically encoded alpha-amino acids: alanine (Ala or A), arginine (Arg or R), asparagine (Asn or N), aspartic acid (Asp or D), cysteine (Cys or C), glutamine (Gln or Q), glutamic acid (Glu or E), glycine (Gly or G), histidine (His or H), isoleucine (Ile or I), leucine (Leu or L), lysine (Lys or K), methionine (Met or M), phenylalanine (Phe or F), proline (Pro or P), serine (Ser or S), threonine (Thr or T), tryptophan (Trp or W), tyrosine (Tyr or Y), and valine (Val or V). In cases where "X" residues are undefined, these should be defined as "any amino acid." The structures of these twenty natural amino acids are shown in, e.g., Stryer et al., Biochemistry, 5th ed., Freeman and Company (2002). Additional amino acids, such as selenocysteine and pyrrolysine, can also be genetically coded for (Stadtman (1996) "Selenocysteine," Annu Rev Biochem. 65:83-100 and Ibba et al. (2002) "Genetic code: introducing pyrrolysine," Curr Biol. 12(13):R464-R466). The term "amino acid" also includes unnatural amino acids, modified amino acids (e.g., having modified side chains and/or backbones), and amino acid analogs. See, e.g., Zhang et al. (2004) "Selective incorporation of 5-hydroxytryptophan into proteins in mammalian cells," Proc. Natl. Acad. Sci. U.S.A. 101(24):8882-8887, Anderson et al. (2004) "An expanded genetic code with a functional quadruplet codon" Proc. Natl. Acad. Sci. U.S.A. 101(20):7566-7571, Ikeda et al. (2003) "Synthesis of a novel histidine analogue and its efficient incorporation into a protein in vivo," Protein Eng. Des. Sel. 16(9):699-706, Chin et al. (2003) "An Expanded Eukaryotic Genetic Code," Science 301(5635):964-967, James et al. (2001) "Kinetic characterization of ribonuclease S mutants containing photoisomerizable phenylazophenylalanine residues," Protein Eng. Des. Sel. 14(12):983-991, Kohrer et al. (2001) "Import of amber and ochre suppressor tRNAs into mammalian cells: A general approach to site-specific insertion of amino acid analogues into proteins," Proc. Natl. Acad. Sci. U.S.A. 98(25):14310-14315, Bacher et al. (2001) "Selection and Characterization of Escherichia coli Variants Capable of Growth on an Otherwise Toxic Tryptophan Analogue," J. Bacteriol. 183(18):5414-5425, Hamano-Takaku et al. (2000) "A Mutant Escherichia coli Tyrosyl-tRNA Synthetase Utilizes the Unnatural Amino Acid Azatyrosine More Efficiently than Tyrosine," J. Biol. Chem. 275(51):40324-40328, and Budisa et al. (2001) "Proteins with {beta}-(thienopyrrolyl)alanines as alternative chromophores and pharmaceutically active amino acids," Protein Sci. 10(7):1281-1292. To further illustrate, an amino acid is typically an organic acid that includes a substituted or unsubstituted amino group, a substituted or unsubstituted carboxy group, and one or more side chains or groups, or analogs of any of these groups. Exemplary side chains include, e.g., thiol, seleno, sulfonyl, alkyl, aryl, acyl, keto, azido, hydroxyl, hydrazine, cyano, halo, hydrazide, alkenyl, alkynl, ether, borate, boronate, phospho, phosphono, phosphine, heterocyclic, enone, imine, aldehyde, ester, thioacid, hydroxylamine, or any combination of these groups. Other representative amino acids include, but are not limited to, amino acids comprising photoactivatable cross-linkers, metal binding amino acids, spin-labeled amino acids, fluorescent amino acids, metal-containing amino acids, amino acids with novel functional groups, amino acids that covalently or noncovalently interact with other molecules, photocaged and/or photoisomerizable amino acids, radioactive amino acids, amino acids comprising biotin or a biotin analog, glycosylated amino acids, other carbohydrate modified amino acids, amino acids comprising polyethylene glycol or polyether, heavy atom substituted amino acids, chemically cleavable and/or photocleavable amino acids, carbon-linked sugar-containing amino acids, redox-active amino acids, amino thioacid containing amino acids, and amino acids comprising one or more toxic moieties.

The term "protein" refers to a polypeptide chain (amino acid sequence) as a product of the ribosomal translation process, wherein the polypeptide chain has undergone posttranslational folding processes resulting in three-dimensional protein structure. The term "protein" also encompasses polypeptides with one or more posttranslational modifications such as (but not limited to) glycosylation, phosphorylation, acetylation and ubiquitination.

Any protein as disclosed herein, particularly recombinantly produced protein as disclosed herein, may in a specific embodiment comprise a "protein tag" which is a peptide sequence genetically grafted onto the recombinant protein. A protein tag may comprise a linker sequence with a specific protease claeavage site to facilitate removal of the tag by proteolysis. As a specific embodiment, an "affinity tag" is appended to a target protein so that the target can be purified from its crude biological source using an affinity technique. For example, the source can be a transformed host organism expressing the target protein or a culture supernatant into which the target protein was secreted by the transformed host organism. Specific embodiments of an affinity tag include chitin binding protein (CBP), maltose binding protein (MBP), and glutathione-S-transferase (GST). The poly(His) tag is a widely-used protein tag which facilitates binding to certain metal chelating matrices.

The term "chimeric protein", "fusion protein" or "fusion polypeptide" refers to a protein whose amino acid sequence represents a fusion product of subsequences of the amino acid sequences from at least two distinct proteins. A fusion protein typically is not produced by direct manipulation of amino acid sequences, but, rather, is expressed from a "chimeric" gene that encodes the chimeric amino acid sequence.

The term "recombinant" refers to an amino acid sequence or a nucleotide sequence that has been intentionally modified by recombinant methods. By the term "recombinant nucleic acid" herein is meant a nucleic acid, originally formed in vitro, in general, by the manipulation of a nucleic acid by endonucleases, in a form not normally found in nature. Thus an isolated, mutant DNA polymerase nucleic acid, in a linear form, or an expression vector formed in vitro by ligating DNA molecules that are not normally joined, are both considered recombinant for the purposes of this invention. It is understood that once a recombinant nucleic acid is made and reintroduced into a host cell, it will replicate non-recombinantly, i.e., using the in vivo cellular machinery of the host cell rather than in vitro manipulations; however, such nucleic acids, once produced recombinantly, although subsequently replicated non-recombinantly, are still considered recombinant for the purposes of the invention. A "recombinant protein" or "recombinantly produced protein" is a protein made using recombinant techniques, i.e., through the expression of a recombinant nucleic acid as depicted above.

A nucleic acid is "operably linked" when it is placed into a functional relationship with another nucleic acid sequence. For example, a promoter or enhancer is operably linked to a coding sequence if it affects the transcription of the sequence; or a ribosome binding site is operably linked to a coding sequence if it is positioned so as to facilitate translation.

The term "host cell" refers to both single-cellular prokaryote and eukaryote organisms (e.g., mammalian cells, insect cells, bacteria, yeast, and actinomycetes) and single cells from higher order plants or animals when being grown in cell culture.

The term "vector" refers to a piece of DNA, typically double-stranded, which may have inserted into it a piece of foreign DNA. The vector or may be, for example, of plasmid origin. Vectors contain "replicon" polynucleotide sequences that facilitate the autonomous replication of the vector in a host cell. Foreign DNA is defined as heterologous DNA, which is DNA not naturally found in the host cell, which, for example, replicates the vector molecule, encodes a selectable or screenable marker, or encodes a transgene. The vector is used to transport the foreign or heterologous DNA into a suitable host cell. Once in the host cell, the vector can replicate independently of or coincidental with the host chromosomal DNA, and several copies of the vector and its inserted DNA can be generated. In addition, the vector can also contain the necessary elements that permit transcription of the inserted DNA into an mRNA molecule or otherwise cause replication of the inserted DNA into multiple copies of RNA. Some expression vectors additionally contain sequence elements adjacent to the inserted DNA that increase the half-life of the expressed mRNA and/or allow translation of the mRNA into a protein molecule. Many molecules of mRNA and polypeptide encoded by the inserted DNA can thus be rapidly synthesized.

The terms "nucleic acid" or "polynucleotide" can be used interchangeably and refer to a polymer that can be corresponded to a ribose nucleic acid (RNA) or deoxyribose nucleic acid (DNA) polymer, or an analog thereof. This includes polymers of nucleotides such as RNA and DNA, as well as synthetic forms, modified (e.g., chemically or biochemically modified) forms thereof, and mixed polymers (e.g., including both RNA and DNA subunits). Exemplary modifications include methylation, substitution of one or more of the naturally occurring nucleotides with an analog, internucleotide modifications such as uncharged linkages (e.g., methyl phosphonates, phosphotriesters, phosphoamidates, carbamates, and the like), pendent moieties (e.g., polypeptides), intercalators (e.g., acridine, psoralen, and the like), chelators, alkylators, and modified linkages (e.g., alpha anomeric nucleic acids and the like). Also included are synthetic molecules that mimic polynucleotides in their ability to bind to a designated sequence via hydrogen bonding and other chemical interactions. Typically, the nucleotide monomers are linked via phosphodiester bonds, although synthetic forms of nucleic acids can comprise other linkages (e.g., peptide nucleic acids as described in Nielsen et al. (Science 254:1497-1500, 1991). A nucleic acid can be or can include, e.g., a chromosome or chromosomal segment, a vector (e.g., an expression vector), an expression cassette, a naked DNA or RNA polymer, the product of a polymerase chain reaction (PCR), an oligonucleotide, a probe, and a primer. A nucleic acid can be, e.g., single-stranded, double-stranded, or triple-stranded and is not limited to any particular length. Unless otherwise indicated, a particular nucleic acid sequence comprises or encodes complementary sequences, in addition to any sequence explicitly indicated.

The term "glycosylation" denotes the chemical reaction of covalently coupling a glycosyl residue to an acceptor group. One specific acceptor group is a hydroxyl group, e.g. a hydroxyl group of another sugar. "Sialylation" is a specific form of glycosylation wherein the acceptor group is reacted with a sialic acid (= N-acetylneuraminic acid) residue. Such a reaction is typically catalyzed by a sialyltransferase enzyme using cytidine-5'-monophospho-N-acetylneuraminic acid as donor compound or co-substrate.

The term "glycosylation" denotes the chemical reaction of covalently coupling a glycosyl residue to an acceptor group. One specific acceptor group is a hydroxyl group, e.g. a hydroxyl group of another sugar. "Sialylation" is a specific form of glycosylation wherein the acceptor group is reacted with a sialic acid (= N-acetylneuraminic acid) residue. Such a reaction is typically catalyzed by a sialyltransferase enzyme using cytidine-5'-monophospho-N-acetylneuraminic acid as donor compound or co-substrate.

"Sialylation" is a specific embodiment of a result of glycosyltransferase enzymatic activity (sialyltransferase enzymatic activity in the particular case), under conditions permitting the same. Generally, the skilled person appreciates that the aqueous buffer in which a glycosyltransferase enzymatic reaction can be performed (= "permitting glycosyltransferase enzymatic activity") needs to be buffered using a buffer salt such as Tris, MES, phosphate, acetate, or another buffer salt specifically capable of buffering in the pH range of pH 6 to pH 8, more specifically in the range of pH 6 to pH 7, even more specifically capable of buffering a solution of about pH 6.5. The buffer may furher contain a neutral salt such as but not limited to NaCl. Further, in particular embodiments the skilled person may consider adding to the aqueous buffer a salt comprising a divalent ion such as Mg²⁺ or Mn²⁺, e.g. but not limited to MgCl₂ and MnCl₂. Conditions permitting glycosyltransferase enzymatic activity known to the art include ambient (room) temperature, but more generally temperatures in the range of 0°C to 40°C, particularly 10°C to 30°C, particularly 20°C.

The term "glycan" refers to a poly- or oligosaccharide, i.e. to a multimeric compound which upon acid hydrolysis yields a plurality of monosachharides. A glycoprotein comprises one or more glycan moieties which are covalently coupled to side groups of the polypeptide chain, typically via asparagine or arginine ("N-linked glycosylation") or via serine or threonine ("O-linked glycosylation").

The use of glycosyltransferases for enzymatic synthesis of complex glycan structures is an attractive approach to obtain complex bioactive glycoproteins. E.g. Barb et al. Biochemistry 48 (2009) 9705-9707 prepared highly potent sialylated forms of the Fc fragment of immunoglobulin G using isolated human ST6Gal-I. However, growing interest in the therapeutic application of glycoproteins leads to an increasing demand of glycosyltransferases including sialyltransferases. Different strategies to increase or modify the sialylation of glycoproteins were described by Bork K. et al. J. Pharm. Sci. 98 (2009) 3499-3508. An attractive strategy is sialylation *in vitro* of recombinantly produced proteins (such as but not limited to immunoglobulins and growth factors), particularly therapeutic proteins. To this end, several research groups described expression of sialyltransferases in transformed organisms and purification of the recombinantly produced sialyltransferases. As glycosyltransferases of prokaryotic origin usually do not act on complex glycoproteins (e.g. antibodies), sialyltransferases from mammalian origin were studied with preference.

Particular glycoproteins subject to the disclosures and all aspects of the present document and the aspects and embodiments herein comprise without limitation cell surface glycoproteins and glycoproteins present in soluble form in serum ("serum glycoprotein"), the glycoproteins particularly being of mammalian origin. A "cell surface glycoprotein" is understood to be glycoprotein of which a portion is located on and bound to the surface of a membrane, by way of a membrane anchor portion of the surface glycoprotein's polypeptide chain, wherein the membrane is part of a biological cell. The term cell surface glycoprotein also encompasses isolated forms of the cell surface glycoprotein as well as soluble fragments thereof which are separated from the membrane anchor portion, e.g. by proteolytic cleavage or by recombinant production of such soluble fragments. A "serum glycoprotein" is understood as a glycoprotein being present in serum, i.e. a blood protein present in the non-cellular portion of whole blood, e.g. in the supernatant following sedimentation of cellular blood components. Without limitation, a specifically regarded and embodied serum glycoprotein is an immunoglobulin. Particular immunoglobulins mentioned in here belong to the IgG group (characterized by Gamma heavy chains), specifically any of four the IgG subgroups. For the disclosures, aspects and embodiments herein the term "serum glycoprotein also encompasses a monoclonal antibody; monoclonal antibodies artificially are well known to the art and can be produced e.g. by hybridoma cells or recombinantly using transformed host cells. A further serum specific glycoprotein is a carrier protein such as serum albumin, a fetuin, or another glycoprotein member of the superfamily of histidine-rich glycoproteins of which the fetuins are members. Further, without limitation, a specifically regarded and embodied serum glycoprotein regarding all disclosures, aspects and embodiments herein is a glycosylated protein signaling molecule. A particular molecule of this group is erythropoietin (EPO).

For *in vitro* engineering of glycoproteins glycosyltransferases can be used as an efficient tool (Weijers 2008). Glycosyltransferases of mammalian origin are compatible with glycoproteins as substrates whereas bacterial glycosyltransferases usually modify simpler substrates like oligosaccharides. For this reason synthetic changes in the glycan moieties of glycoproteins are advantageously made using mammalian glycosyltransferases as tools of choice. However, for a large scale application of glycosyltransferases in glycoengineering availability of suitable enzymes in large (i.e. industrial) quantities is required. The invention described herein particularly provides several variants with truncation deletions. Particularly and surprisingly Δ108 hST6Gal-I exhibits sialylating hST6Gal-I enzyme activity.

Each truncation variant described herein is given a "delta" (= "Δ") designation indicating the number of the last amino acid position of the respective truncation deletion, counted from the N-Terminus of the wild-type hST6Gal-I polypeptide according to SEQ ID NO:1 Several different N-terminal truncation variants, particularly Δ108 hST6Gal-I (amino acid sequence shown in SEQ ID NO:7) were studied in more detail.

Several human glycosyltransferases, including hST6Gal-I were successfully expressed in soluble form in the methylotrophic yeast *Pichia pastoris.* However, only low quantities of proteins were expressed, e.g. ST6Gal-I: 0.3 units/L (Malissard et al. Biochem. Biophys. Res. Commun. 267 (2000) 169-171). Several authors describe the use of alternative expression systems to improve the expression rate and solubility of recombinant ST6Gal-I. For example, a FLAG-tagged recombinant ST6Gal-I was expressed in silkworm larvae, however again with a low yield (Ogata M. et al. BMC Biotechnol. 9 (2009) 54). Another group expressed in *E. coli* a soluble form of ST6Gal-I lacking the cytosolic and membrane regions, and fused with a maltose-binding protein (MBP) tag. However, after purification of the target enzyme only small quantities were obtained (Hidari, et al. Glycoconjugate Journal 22 (2005) 1-11. US 5,032,519 describes expression and isolation of a truncated rat ST6Gal-I in mammalian and insect cells. The enzyme contains amino acids 58-403 of the naturally occurring (wild-type) gene, including the major part of the stem region.

A first aspect as disclosed herein is a variant (= mutant allele of a) mammalian glycosyltransferase, wherein the polypeptide of the variant comprises an N-terminally truncated amino acid sequence of the wild-type mammalian glycosyltransferase (reference), the truncation comprising the amino acid sequence motif of SEQ ID NO:2, and wherein the variant exhibits glycosyltransferase activity. Thus, one of the newly discovered variant mammalian glycosyltransferase enzymes is truncated by a deletion from the N-terminus, wherein the deletion comprises the motif of SEQ ID NO:2 ("QVWxKDS"). Surprisingly, this variant retains glycosyltransferase activity. In one embodiment of all aspects as reported herein, the variant is a deletion mutant of a wild-type mammalian glycosyltransferase polypeptide, wherein the deletion comprises a contiguous N-terminal portion (truncation) including an amino acid sequence comprising the conserved motif "QVWxKDS" wherein in the conserved motif "x" designates a single variable amino acid, and wherein the deletion mutant retains glycosyltransferase activity. In one specific embodiment of all aspects as reported herein, "x" designates asparagine (= Asn or N).

In one embodiment of all aspects as reported herein, the glycosyltransferase, i.e. the activity activity of the variant glycosyltransferase enzyme as disclosed herein, catalyzes a chemical reaction which includes a transfer of a 5-N-acetylneuraminic acid (= Neu5Ac) residue from a donor compound to an acceptor group. In one embodiment of all aspects as reported herein, the glycosyltransferase is a sialyltransferase. In a particular embodiment of all aspects as reported herein, the acceptor group being the target of the transfer of the Neu5Ac residue is the galactosyl residue of a terminal β-D-galactosyl-1,4-N-acetyl-β-D-glucosamine (= Galβ1,4GlcNAc) in a glycan moiety of a glycoprotein or of a glycolipid. In a particular embodiment of all aspects as reported herein, the donor compound is cytidine-5'-monophospho-N-acetylneuraminic acid (= CMP-Neu5Ac or CMP-NANA) or a functional equivalent thereof. A particular functional equivalent in this regard is CMP-9-fluoro-NANA. More generally, a functional equivalent of CMP-Neu5Ac is capable of serving as a co-substrate for a sialyltransferase by providing an activated sugar or sugar derivative, wherein the sugar or sugar derivative is transferred to the acceptor group by enzymatic catalysis of the sialyltransferase.

In the particular case of CMP-Neu5Ac as the donor compound, and in one embodiment of all aspects as reported herein, the glycosyltransferase activity catalyzes a chemical reaction which includes reacting the Neu5Ac residue from the donor compound CMP-Neu5Ac with the hydroxyl group at the C6 position in the galactosyl residue of Galβ1,4GlcNAc, wherein N-acetylneuraminyl-α2,6-β-D-galactosyl-1,4-N-acetyl-β-D-glucosamine (= Neu5Acα2,6Galβ1,4GlcNAc) is formed.

In one embodiment of all aspects as reported herein, the wild-type reference molecule of the variant mammalian glycosyltransferase as disclosed herein is of natural origin, i.e. it is a naturally occurring mammalian glycosyltransferase, specifically a naturally occurring sialyltransferase. A specific embodiment thereof is an enzyme of human origin, particularly a human sialyltransferase, and more specifically a sialyltransferase capable of catalyzing an α2,6 sialylation. Even more specifically, the wild-type reference molecule of the variant mammalian glycosyltransferase as disclosed herein is a human β-galactoside-α2,6-sialyltransferase (hST6Gal). In one embodiment of all aspects as reported herein, the variant mammalian glycosyltransferase is derived from the wild-type reference molecule of SEQ ID NO:1. In a specific embodiment thereof the variant comprises an amino acid sequence of the wild-type mammalian glycosyltransferase according to SEQ ID NO:1, truncated by a deletion from the N-terminus, and the deletion comprises the motif of SEQ ID NO:2, wherein X is an asparagine (N).

Whereas deletions of (i) the short N-terminal cytoplasmic tail, (ii) the transmembrane domain or (iii) the stem region were previously found to be compatible with sialyltransferase activity, truncations affecting the catalytic domain completely abolish the enzymatic activity. In contrast to the present disclosure, the state of the art teaches that certain less extensive truncations of N-terminal amino acids of human ST6Gal-I appear to be critical for the enzymatic activity. A previous report discloses that in the hST6Gal-I amino acid sequence the boundary between stem domain and catalytic domain is located between the amino acid at position 86 and the amino acid at position 104 (Chen C & Colley K.J. Glycobiology 10 (2000) 531-538). Experiments were made by progressive N-terminal truncation to determine the minimal size of a catalytically active hST6Gal-I. As could be expected, variants with deletions covering the first 80 amino acids were enzymatically active. However, truncation of hundred amino acids abolished enzymatic activity (Legaigneur et al. J. Biol. Chem. 276 (2001) 21608-21617). In another publication, an inactive enzyme yielded by a truncation comprising residues 94-100 in the amino acid sequence of hST6Gal-I was found; from the result it was concluded the the amino acid residues at positions 94-100 were crucial for enzymatic activity when deleted. Residues 94-100 in the amino acid sequence of the hST6Gal-I polypeptide correspond to the QVWxKDS motif (Donadio et al., *supra*).

Specific embodiments of all aspects as reported herein include a variant hST6Gal-I, wherein the amino acid sequence of the wild-type reference polypeptide is SEQ ID NO:1 and the variant hST6Gal-I is characterized by a truncation selected from the group consisting of (i) position 1 to position 100 of SEQ ID NO:1, (ii) position 1 to position 101 of SEQ ID NO:1, (iii) position 1 to position 102 of SEQ ID NO:1, (vi) position 1 to position 103 of SEQ ID NO:1, (v) position 1 to position 104 of SEQ ID NO:1, (vi) position 1 to position 105 of SEQ ID NO:1, (vii) position 1 to position 106 of SEQ ID NO:1, (viii) position 1 to position 107 of SEQ ID NO:1, and (ix) position 1 to position 108 of SEQ ID NO:1.

Thus, further specific embodiments of all aspects as reported herein include a polypeptide with an amino acid sequence selected from the group consisting of (i) position 101 to position 406 of SEQ ID NO:1, (ii) position 102 to position 406 of SEQ ID NO:1, (iii) position 103 to position 406 of SEQ ID NO:1, (vi) position 104 to position 406 of SEQ ID NO:1, (v) position 105 to position 406 of SEQ ID NO:1, (vi) position 106 to position 406 of SEQ ID NO:1, (vii) position 107 to position 406 of SEQ ID NO:1, (viii) position 108 to position 406 of SEQ ID NO:1, and (ix) position 109 to position 406 of SEQ ID NO:1.

For *in vitro* engineering of glycoproteins glycosyltransferases can be used as an efficient tool (Weijers 2008). Glycosyltransferases of mammalian origin are compatible with glycoproteins as substrates whereas bacterial glycosyltransferases usually modify simpler substrates like oligosaccharides. For this reason synthetic changes in the glycan moieties of glycoproteins are advantageously made using mammalian glycosyltransferases as tools of choice. However, for a large scale application of glycosyltransferases in glycoengineering availability of suitable enzymes in large (i.e. industrial) quantities is required. The invention described herein particularly provides a protein with hST6Gal-I enzyme activity which can be used for *in vitro* sialylisation of target glycoproteins with one or more accessible galactosyl substrate moiety/moieties. Suitable targets include asialoglycoproteins, i.e. glycoproteins from which sialic acid residues have been removed by the action of sialidases.

While expressing wild-type hST6Gal-I in the methylotrophic yeast *Pichia pastoris* and having targeted the expressed polypeptide to the secretory pathway of the host organism, different truncated variants of recombinantly produced hST6Gal-I were observed. Generally, hST6Gal-I derived proteins were chromatographically purified and analyzed, particularly by means of mass spectrometry and by way of determining the amino acid sequence from the N-terminus (Edman degradation). By these means truncations, particularly N-terminal truncations of hST6Gal-I were characterized in detail.

Several remarkable truncation variants were identified in the supernatants of transformed *Pichia* strains. The variants could possibly result from site-specific proteolytic cleavage during the course of secretion from the yeast cells, or result from endoproteolytic cleavage by one or more extracellular protease(s) present in the supernatant of cultured *Pichia* strains.

Each identified truncation variant was given a "delta" (= "Δ") designation indicating the number of the last amino acid position of the respective truncation deletion, counted from the N-Terminus of the wild-type hST6Gal-I polypeptide according to SEQ ID NO:1 Five different N-terminal truncation variants, Δ27 (SEQ ID NO:3), Δ48 (SEQ ID NO:4), Δ62 (SEQ ID NO:5), Δ89 (SEQ ID NO:6), and Δ108 (SEQ ID NO:7) of hST6Gal-I were studied in more detail.

Surprisingly, the truncation variant Δ108 of hST6Gal-I (i.e. a variant hST6Gal-I protein with a polypeptide lacking the amino acids at positions 1-108 which are present in the corresponding wild-type polypeptide) was found to be enzymatically active; that is to say the Δ108 truncation variant of hST6Gal-I is capable of catalyzing transfer of a Neu5Ac residue to the C6 hydroxyl group of a free galactosyl residue being part of terminal Galβ1,4GlcNAc in a glycan or an antenna of a glycan, thereby forming in the glycan a terminal sialic acid residue α2→6 linked to the galactosyl residue of the Galβ1,4GlcNAc moiety. Furthermore, the Δ108 truncation variant of hST6Gal-I is suitable for glycoengineering applications to synthetically change the composition of glycan moieties of glycoproteins. Moreover, the Δ108 truncation variant of hST6Gal-I is well suited for recombinant expression in different host organisms, thereby allowing production of this enzyme in high amounts and at reasonable cost.

It is further remarkable that a Δ108 N-terminal truncation variant of hST6Gal-I (see "batch 5", batch 7" of Table 1 in Example 14) was more active, i.e. by a factor of about 20 times more active, compared to a preparation which contained a Δ114 truncation variant (see "batch 3" in Table 1 in Example 14). Thus, removal of the respective N-terminal portions of hST6Gal-I nevertheless left an enzyme moiety capable of catalyzing the sialylation reaction. However, it could not entirely be excluded that the preparations in which the Δ114 truncation variant was detected additionally contained traces of the Δ108 or another enzymatically active variant of hST6Gal-I which could be responsible for the observed residual activity. If this had been the case the Δ114 variant could be inactive.

Expression vectors were constructed for expression of hST6Gal-I wild-type protein as well as of selected truncation variants in various host organisms including prokaryotes such as *E. coli* and *Bacillus sp.,* yeasts such as *Saccharomyces cerevisiae* and *Pichia pastoris,* and mammalian cells such as CHO cells and HEK cells. Vectors with expression constructs not only for the Δ108 truncation variant of hST6Gal-I were made but also for the other four identified truncated forms (Δ27ST6, Δ48ST6, Δ62ST6 and Δ89ST6) of human ST6Gal-I. To facilitate purification of the recombinantly expressed target proteins, the truncation variant polypeptides encoded by the constructs usually included a N-terminal His-tag.

In a particular series of experiments, expression constructs were inserted into vectors for propagation in *Pichia pastoris* strain KM71H. Expression typically was controlled by an inducible promoter such as the AOX1 promoter. His-tagged truncation variants were additionally fused to a leader peptide capable of targeting the expressed primary translation product to the secretory pathway of the transformed host. Posttranslational processing thus included secretion of the respective His-tagged truncation variant into the surrounding medium while the leader peptide was cleaved off by an endoprotease of the secretion machinery.

Transformed *Pichia* cells were typically cultured in a liquid medium. After induction of expression, the transformed cells were cultured for a certain time to produce the respective target protein. Following the termination of the culturing step, the cells and other insoluble materials present in the culture were separated from the supernatant. The truncation variants of hST6Gal-I in the cleared supernatants were analyzed.

First experiments in *Pichia* were conducted with N-terminally His-tagged wild-type hST6Gal-I as target protein. However, attempts to purify the enzyme from the supernatant failed when a chromatography column loaded with a Ni-chelating affinity matrix was used, as the active enzyme was not retained on the column but was found in the flow-through. Similar results were subsequently obtained with N-terminally His-tagged truncation variants of hST6Gal-I. Purification of the enzymes (wild type and variants) using a cation exchange resin nevertheless resulted in highly enriched enzyme preparations. But this purification procedure generally appeared to affect the activity of the enzymes negatively.

Surprisingly, when characterized by SDS gel electrophoresis all hST6Gal-I proteins purified by cation exchange chromatography showed an apparent molecular weight of about 36 kDa. In line with the negative results of the attempts to use Ni-chelating affinity matrix for purification, N-terminal sequencing and mass spectrometry confirmed the absence of N-terminal His tags. For the purified samples of different truncated hST6Gal-I proteins secreted from transformed *Pichia,* several N-terminal sequences were found corresponding to variants with Δ108, Δ112, and Δ114 truncations. The wide spectrum of proteolytic products seems to indicate several truncation mechanisms, most likely as a result of proteolytic digestion by more than one type of protease.

Further analysis of the samples of the truncated hST6Gal-I proteins revealed that removal of a contiguous N-terminal portion with more than 112 amino acids from the polypeptide significantly reduced the enzymatic activity of hST6Gal-I. This finding was very much in contrast to a truncation of 108 amino acids from the N-terminus which appears to be an active hST6Gal-I enzyme. However, in comparison to the Δ89 truncation variant the Δ108 enzyme displays a relative activity of about 50%.

From one Pichia pastoris clone designed to express and secrete the N-terminally His-tagged Δ62 ST6Gal-I construct a highly active and hST6Gal-I enzyme with a homogeneous size was isolated and analyzed in more detail. Again, the enzyme in the supernatant lacked the His-tag. The N-terminal sequence was determined to be "LQKIWKNYLS" which corresponds to a Δ108 variant of hST6Gal-I. The original primary translation product was a polypeptide with an N-terminal signal peptide portion, followed by a His-tag, followed by the amino acid sequence of Δ62 hST6Gal-I. Because the Δ108 truncation variant was found in the supernatant it was concluded that only after completion of the secretory process the further truncation from Δ62 to Δ108 did occur. Had this truncation taken place prior to secretion, the Δ108 polypeptide would not have been secreted due to a premature loss of the signal peptide portion. Thus, the finding was interpreted that the His-tagged a Δ62 variant of hST6Gal-I was truncated by proteolytic digestion outside the cellular compartment.

A specific aspect of the disclosure herein is the use a variant mammalian glycosyltransferase, particularly the Δ108 variant of hST6Gal-I, for transferring a 5-N-acetylneuraminic acid residue from a donor compound to a hydroxyl group at the C6 position in the galactosyl residue of a terminal β-D-galactosyl-1,4-N-acetyl-β-D-glucosamine of a glycan moiety of a target glycoprotein with an acceptor group. An example therefor is a monoclonal antibody of the immunoglobulin G class. In a specific embodiment of all aspects as disclosed herein the target molecule is free of α2,6 sialylated terminal antennal (acceptor) residues. One out of several ways to arrive at such a target molecule is to remove any terminal sialyl residues with an enzyme having glycosidase, and specifically sialidase activity. Thus, making use of such an "asioalo" target protein which however retains an acceptor site for sialylation, the present disclosure, particularly the above use and method enables the skilled person to prepare sialylated target molecules selected from a glycoprotein and a glycolipid.

As exemplified herein, the Δ108 variant was active in sialylation experiments using a recombinantly produced human monoclonal IgG4 antibody as a complex target (substrate); similar findings were obtained using as a sialylation target a human IgGl monoclonal antibody. Expression constructs encoding the Δ108 variant were made, cloned in Pichia pastoris KM71H, and expressed in high quantities. The recombinantly expressed protein was secreted into the liquid growth medium and purified therefrom. In addition, expression constructs of the Δ108 variant were introduced into HEK 293 cells, transiently expressed, secreted and purified. Analysis confirmed that this variant expressed in HEK cells was also enzymatically active, i.e. capable of sialylating monoclonal antibodies.

The detection of a truncated, but enzymatically active vartiant of human ST6Gal-I (Δ108 ST6Gal-I) was a new and surprising finding, and a contribution to the present knowledge which suggests that deletions of more than 100 amino acids completely abolish the enzymatic activity of hST6Gal-I (Chen& Colley, 2000; Legaigneur et al., (2001) J. Biol. Chem., 276, 21608-17; Donadio et al. 2003).

Another aspect as disclosed herein is a fusion polypeptide comprising a polypeptide of a variant mammalian glycosyltransferase as disclosed herein.

Yet, another aspect as disclosed herein is a nucleotide sequence encoding a variant mammalian glycosyltransferase as disclosed herein.

Yet, another aspect as disclosed herein is an expression vector comprising a target gene and sequences facilitating expression of the target gene in a host organism transformed with the expression vector, wherein the target gene comprises a nucleotide sequence as disclosed herein.

Yet, another aspect as disclosed herein is a transformed host organism, wherein the host organism is transformed with an expression vector as disclosed herein. With particular advantage, Human Embryonic Kidney 293 (HEK) cells can be used to practice the teachings as disclosed in here. A particular advantage of these cells is that they are very suited targets for transfection followed by subsequent culture. Thus, HEK cells can be efficiently used to produce target proteins by way of recombinant expression and secretion. Nevertheless, HeLa, COS and Chinese Hamster Ovary (CHO) cells are well-known alternatives and are included herein as specific embodiments of all aspects as disclosed herein.

Yet, another aspect as disclosed herein is a method to produce recombinantly a variant mammalian glycosyltransferase, the method comprising the step of expressing in a host organism transformed with an expression vector a nucleotide sequence encoding a variant mammalian glycosyltransferase as disclosed herein, wherein a polypeptide is formed, thereby producing variant mammalian glycosyltransferase.

The following items further provide specific aspects of the disclosure, and specific embodiments to practice the teachings provided herein.
1. A variant mammalian glycosyltransferase, wherein the polypeptide of the variant comprises an N-terminally truncated amino acid sequence of the wild-type mammalian glycosyltransferase, the truncation comprising the amino acid sequence motif of SEQ ID NO:2, and wherein the variant exhibits glycosyltransferase activity.
2. The variant according to item 1, wherein the glycosyltransferase activity catalyzes a chemical reaction which includes a transfer of a 5-N-acetylneuraminic acid (= Neu5Ac, NANA) residue from a donor compound to an acceptor group.
3. The variant according to item 2, wherein the acceptor group is the galactosyl residue of a terminal β-D-galactosyl-1,4-N-acetyl-β-D-glucosamine (= Galβ1,4GlcNAc) in a glycan moiety of a glycoprotein or of a glycolipid.
4. The variant according to any of the items 2 and 3, wherein the donor compound is cytidine-5'-monophospho-N-acetylneuraminic acid (= CMP-Neu5Ac) or a functional equivalent thereof.
5. The variant according to item 4, wherein the chemical reaction catalyzed by the glycosyltransferase activity includes reacting the Neu5Ac residue from the donor compound CMP-Neu5Ac with the hydroxyl group at the C6 position in the galactosyl residue of β-D-galactosyl-1,4-N-acetyl-β-D-glucosamine, wherein N-acetylneuraminyl-α2,6-β-D-galactosyl-1,4-N-acetyl-β-D-glucosamine (Neu5Acα2,6Galβ1,4GlcNAc) is formed.
6. The variant according to any of the items 1 to 5, wherein the wild-type mammalian glycosyltransferase is of natural origin.
7. The variant according to item 6, wherein the wild-type mammalian glycosyltransferase is of human origin.
8. The variant according to item 7, wherein the wild-type mammalian glycosyltransferase is a human β-galactoside-α-2,6-sialyltransferase.
9. The variant according to item 8, wherein the polypeptide of the variant comprises an N-terminally truncated amino acid sequence of the wild-type mammalian glycosyltransferase according to SEQ ID NO:1, the truncation comprising the amino acid sequence motif of SEQ ID NO:2, wherein X is asparagine (N).
10. The variant according to item 9, wherein the truncation is a sequence selected from the group consisting of (i) position 1 to position 100 of SEQ ID NO:1, (ii) position 1 to position 101 of SEQ ID NO:1, (iii) position 1 to position 102 of SEQ ID NO:1, (vi) position 1 to position 103 of SEQ ID NO:1, (v) position 1 to position 104 of SEQ ID NO:1, (vi) position 1 to position 105 of SEQ ID NO:1, (vii) position 1 to position 106 of SEQ ID NO:1, (viii) position 1 to position 107 of SEQ ID NO:1, and (ix) position 1 to position 108 of SEQ ID NO:1.
11. The variant according to item 9, wherein the deletion from the N-terminus is the sequence of position 1 to position 108 of SEQ ID NO:1.
12. The variant according to any of the items 1 to 11, wherein the N-terminus or C-terminus of the polypeptide of the variant is fused to an affinity tag.
13. The variant according to item 12, wherein the affinity tag comprises four, five, six or more consecutive histidine residues.
14. The variant according to any of the items 12 and 13, wherein a peptidase cleavage site is located between the affinity tag and the N-terminus or C-terminus of the polypeptide of the variant.
15. The variant according to any of the items 1 to 14, wherein the polypeptide of the variant further comprises a N-terminal methionine residue.
16. A fusion polypeptide comprising the polypeptide of a variant mammalian glycosyltransferase according to any of the items 1 to 15.
17. A nucleotide sequence encoding the polypeptide of a variant mammalian glycosyltransferase according to any of the items 1 to 15.
18. A nucleotide sequence encoding the polypeptide of a fusion polypeptide comprising the polypeptide of a variant mammalian glycosyltransferase according to any of the items 1 to 15.
19. An expression vector comprising a target gene operably linked to sequences facilitating expression of the target gene in a host organism transformed with the expression vector, wherein the target gene comprises a nucleotide sequence according to item 17 or item 18.
20. A transformed host organism, wherein the host organism is transformed with an expression vector according to item 19.
21. A method to produce recombinantly a variant mammalian glycosyltransferase, the method comprising the step of expressing in a transformed host organism a nucleotide sequence encoding the variant mammalian glycosyltransferase according to any of the items 1 to 15, wherein a polypeptide is formed, thereby producing the variant mammalian glycosyltransferase.
22. The method according to item 21, wherein the produced enzyme is secreted from the host organism.
23. The method according to any of the items 21 and 22, wherein the host organism is a eukaryotic cell.
24. The method according to item 23, wherein the host organism is selected from a yeast cell and a mammalian cell.
25. The method according to item 24, wherein the host organism is a mammalian cell selected from the group consisting of a HEK cell, a COS cell, a CHO cell, and a HeLa cell.
26. The method according to any of the items 21 to 25, wherein the variant mammalian glycosyltransferase is purified.
27. A variant of human β-galactoside-α-2,6-sialyltransferase I, obtained by a method according to any of the items 21 to 26, wherein the host organism is selected from a *Pichia pastoris* cell, a CHO cell and a HEK cell.
28. Use of a variant mammalian glycosyltransferase according to any of the items 1 to 15, or a variant of human β-galactoside-α-2,6-sialyltransferase I obtained by a method according to any of the items 21 to 26, for transferring a 5-N-acetylneuraminic acid residue from a donor compound to a hydroxyl group at the C6 position in the galactosyl residue of a terminal β-D-galactosyl-1,4-N-acetyl-β-D-glucosamine of a glycan moiety of a monoclonal antibody.
29. A method of sialylating a glycoprotein, comprising the step of contacting in aqueous solution under conditions permissive for glycosyltransferase enzymatic activity the following compounds: (i) a glycoprotein having in a glycan moiety a terminal β-D-galactosyl-1,4-N-acetyl-β-D-glucosamine acceptor group, (ii) the donor compound, (iii) a variant of human β-galactoside-α-2,6-sialyltransferase I wherein the polypeptide of the variant comprises an N-terminally truncated amino acid sequence of the wild-type mammalian glycosyltransferase, the truncation comprising the amino acid sequence motif of SEQ ID NO:2, and wherein the variant exhibits glycosyltransferase activity, thereby forming a N-acetylneuraminyl-α2,6-β-D-galactosyl-1,4-N-acetyl-β-D-glucosamine residue, thereby sialylating the glycoprotein.
30. The method according to item 29, wherein the glycoprotein is an immunoglobulin, and particularly a monoclonal antibody.

The Examples that follow are illustrative of specific embodiments of the disclosure, and various uses thereof. They set forth for explanatory purposes only, and are not to be taken as limiting the disclosure.

### Example 1

### Database search for eukaryotic glycosyltransferases sharing the QVWxKDS consensus motif

The search was performed using publically available databases, particularly the "swissprot" database, and search algorithms, e.g. BLAST (= "Basic Local Alignment Search Tool"), according to the disclosure of Altschul S.F. et al. Nucleic Acids Res. 25 (1997) 3389-3402. The motif QVWxKDS (SEQ ID NO:2) was found in each of the following polypeptide sequences which are presented by way of example:
(a) Sequence ID: sp|Q64685.2|SIAT1_MOUSE; Beta-galactoside alpha-2,6-sialyltransferase I
(b) Sequence ID: sp|P13721.1|SIAT1_RAT; Beta-galactoside alpha-2,6-sialyltransferase I
(c) Sequence ID: sp|P15907.1|SIAT1_HUMAN; Beta-galactoside alpha-2,6-sialyltransferase I

### Example 2

### Cloning and expression of hST6Gal-I

A first series of expression constructs was designed for *Pichia pastoris* as a host organism. Generally, the methods suggested and described in the Invitrogen manuals "*Pichia* Expression Kit" Version M 011102 25-0043 and "pPICZα A, B, and C" Version E 010302 25-0150 were applied. Reference is also made to further vectors, yeast strains and media mentioned therein. Basic methods of molecular biology were applied as described, e.g., in Sambrook, Fritsch & Maniatis, Molecular Cloning, A Laboratory Manual, 3rd edition, CSHL Press, 2001.

In each case, expression of the respective hST6Gal-I construct was under the control of the Pichia pastoris AOX1 promoter which is inducible by methanol.

Each of the constructs was inserted as a cassette into a pPICZαB vector, using the restriction sites of *XhoI* and *NotI.* This way the coding sequence for the signal peptide (nucleotide sequence encoding the α-factor signal peptide from *Saccharomyces cerevisiae*) was fused in-frame with the coding sequence of the His-tagged hST6Gal-I polypeptide sequence (i.e. the full-length hST6Gal-I polypeptide or a variant thereof).

At the junction region between the signal peptide and the His-tag there was a KEX2-like processing site in the precursor polypeptide sequence, i.e. a signal peptidase cleavage site needed to cleave off the signal peptide from the precursor protein during the course of secretion. The N-terminal signal peptide was found suitable to direct each of the the primary translation products to the secretory pathway of the yeast. As a result, the recombinantly expressed hST6Gal-I polypeptides were exported into the liquid culture media in which the recombinant *Pichia pastoris* strains were cultivated.

Codon optimized (for expression in Pichia pastoris) nucleotide sequences encoding truncated variants of hST6Gal-I Δ27, Δ48, Δ62, Δ89, Δ108 and are shown in SEQ ID NOs: 8, 10, 12, 14, and 16, respectively. SEQ ID NOs: 9, 11, 13, 15, and 17, show the his-tagged sequences subject to expression experiments in Pichia pastoris. Culture supernatants from each variant were produced and the hST6Gal-I enzyme variants comprised therein were purified and characterized.

### Example 3

### Truncation variants of hST6Gal-I

Several truncation variants were expressed in *Pichia pastoris* as described technically in Example 2 above.

Figure 1 discloses the amino acid sequence of human β-galactoside-α-2,6-sialyltransferase I (ST6Gal-I, E.C 2.4.99.1; UniProtKB/Swiss-Prot data base entry "P15907"), SEQ ID NO:1, and presents a schematic representation of deletions in ther N-terminal portion of the polypeptide which were characterized in more detail.

For each of the variants, the N-terminally deleted amino acid positions are indicated by "X". The residues of the "QVWNKDS" motif according to position 94-100 of SEQ ID NO:2, wherein X is an asparagine, are underlined.

### Example 4

### Transformation and fermentation of transformed Pichia pastoris

Variants of human ST6Gal-I gene were expressed in *Pichia pastoris* KM71H. The cells were grown in complex glycerol medium at 30°C and pH 5.2 to an OD578 (= optical density measured at a wavelength of 578 nm) of 200. After reduction of the temperature to 20°C the expression of the ST6Gal-I gene in the respective expression cassette was induced by feeding the cells with methanol. At a final OD578 of 400 the culture medium was cooled to 4°C and the cells were separated by centrifugation. The supernatants containing the enzyme variants of ST6Gal-I were stored at -20°C.

### Example 5

### Cloning of pM1MT expression constructs for transient gene expression (TGE) in mammalian host cells

Truncated variant Δ108 of human ST6Gal-I was cloned for transient expression using an Erythropoietin signal peptide sequence (Epo) and a peptide spacer of four amino acids ("APPR"). For the Epo-APPR-Δ108 hST6Gal-I conctruct a codon-optimized cDNAs was synthesized. The natural hST6Gal-I-derived mRNA leader and N-terminal protein sequences were exchanged with the Erythropoetin signal sequence and the "APPR" linker sequence to ensure correct processing of the polypeptide by the secretion machinery of the HEK host cell line. In addition, the expression cassettes feature *Sa*lI and *BamHI* sites for cloning into the multiple cloning site of the pre-digested pM1MT vector fragment (Roche Applied Science). Expression of the hST6Gal-I coding sequence was thereby put under the control of a human cytomegalovirus (CMV) immediate-early enhancer/promoter region; the expression vector further featured an "intron A" for regulated expression and a BGH polyadenylation signal.

Expression of the Epo-APPR-Δ108 hST6Gal-I conctruct (SEQ ID NO:18) in HEK cells, and secretion of Δ108 hST6Gal-I protein into cell supernatant was performed as describes in Example 6.

### Example 6

### Transformation HEK cells and transient expression and secretion

Transient gene expression (TGE) by transfection of plasmid DNA is a rapid strategy to produce proteins in mammalian cell culture. For high-level expression of recombinant human proteins a TGE platform based on a suspension-adapted human embryonic kidney (HEK) 293 cell line was used. Cells were cultured in shaker flasks at 37°C under serum-free medium conditions. The cells were transfected at approx. 2x 10⁶ vc/ml with the pM1MT expression plasmids (0.5 to 1 mg/L cell culture) complexed by the 293-Free™ (Merck) transfection reagent according to the manufacturer's guidelines. Three hours post-transfection, valproic acid, a HDAC inhibitor, was added (final conc. 4 mM) in order to boost the expression (Backliwal et al. (2008), Nucleic Acids Research 36, e96). Each day, the culture was supplemented with 6% (v/v) of a soybean peptone hydrolysate-based feed. The culture supernatant was collected at day 7 post-transfection by centrifugation.

### Example 7

### Test for sialyltransferase enzymatic activity

Enzymatic activity was determined by measuring the transfer of sialic acid to asialofetuin. The reaction mix (0.1 M MES, pH 6.0) contained 2.5 µg of enzyme sample, 5 µL asialofetuin (10 mg/ml) and 4 µL CMP-9-fluoro-NANA (0.2 mM) in a total volume of 51 µL. The reaction mix was incubated at 37°C for 30 minutes. The reaction was stopped by the addition of 10 µL of the inhibitor CTP (10 mM). The reaction mix was loaded onto a PD10 desalting column equilibrated with 0.1 M Tris/HCl, pH 8.5. Fetuin was eluted from the column using the equilibration buffer. The fractions size was 1 mL. The concentration of formed fetuin was determined using a fluorescence spectrophotometer. Excitation wave length was 490 nm, emission was measured at 520 nm. Enzymatic activity was expressed as RFU (relative fluorescence unit).

### Example 8

### SDS gel electrophoresis

Analytical SDS gel electrophoresis was carried out using NuPAGE gels (4-12%, Invitrogen). Samples were stained using SimplyBlue SafeStain (Invitrogen). All procedures were performed according to the recommendations of the manufacturer.

### Example 9

### N-terminal sequencing by Edman degradation

The N-terminal sequences of expressed variants of human ST6Gal-I were analyzed by Edman degradation using reagents and devices obtained from Life Technologies. Preparation of the samples was done as described in the instruction manual of the ProSorb Sample Preparation cartridges (catalog number 401950) and the ProBlott Mini PK/10 membranes (catalog number 01194). For sequencing the Procise Protein Sequencing Platform was used.

### Example 10

### Mass spectrometry

The molecular masses of variants of human ST6Gal-I expressed in Pichia and HEK cells were analyzed in mass spectrometry. Therefore, the glycosylated and deglycosylated forms of human ST6Gal-I were prepared and analyzed using Micromass Q-Tof Ultima and Synapt G2 HDMS devices (Waters UK) and MassLynx V 4.1 software.

### Example 11

### Mass spectrometry of glycosylated human ST6Gal-I enzymes

For mass spectrometry measurement the samples were buffered in electrospray medium (20 % acetonitrile + 1 % formic acid). The buffer exchange was performed with illustra™ MicroSpin™ G-25 columns (GE-Healthcare). 20 µg sialyltransferase variant with a concentration of 1 mg/ml was applied to the pre-equilibrated column and eluated by centrifugation. The resulting eluate was analyzed by electrospray ionization mass spectrometry.

### Example 12

### Mass spectrometry of deglycosylated human ST6Gal-I enzymes

For deglycosylation samples of the sialyltransferase were denatured and reduced. To 100 µg sialyltransferase 45 µL denaturing buffer (6 M guanidinium chloride) and 13 µL TCEP (0.1 mM, diluted in denaturing buffer) were added. Further the appropriate volume of ultrapure water was added, so that the overall concentration of guanidinium chloride is about 4 M. Then the sample was incubated for 1 hour at 37°C. To get rid of denaturing and reducing agent rebuffering was done. Therefore Bio-SpinR 6 Tris columns (Bio Rad) were used, which were pre-equilibrated with ultrapure water. The whole sample was applied onto the column and eluted by centrifugation. To the resulting eluate 5.5 µL of 0.1 U/µl solution of PNGase F was added and incubated at 37°C over night. Afterwards the samples were adjusted to 30% ACN and 1% FA and analyzed by electrospray ionization mass spectrometry.

### Example 13

### Purification of human ST6Gal-I variants recombinantly expressed in and secreted from transformed Pichia pastoris KM71H

Variants of human ST6Gal-I were purified from fermentation supernatants of *Pichia pastoris* KM71H. The purification was essentially carried out by two chromatographic methods. In a first step, two liters of supernatant were centrifuged (15 min, 8500 rpm). After an ultrafiltration step (0.2 µm), the solution was dialyzed against buffer A (20 mM potassium phosphate, pH 6.5) and concentrated. The dialysate was loaded onto a S-Sepharose™ Fast Flow column (5.0 cm x 5.1 cm) equilibrated with buffer A. After washing with 600 mL buffer A, the enzyme was eluted with a linear gradient of 100 mL buffer A and 100 mL of buffer A + 200 mM NaCl, followed by a wash step using 300 mL of buffer A + 200 mM NaCl. Fractions (50 mL) were analysed by an analytical SDS gel (4-12%). The fraction containing ST6Gal-I were pooled and dialysed against buffer C (50 mM MES, pH 6.0). The dialysate was loaded onto a Capto MMC column (1.6 cm x 3.0 cm) equilibrated with buffer C. After washing the column with 150 mL buffer C, the enzyme was eluted with a linear gradient of 60 mL buffer C and 60 mL buffer D (50 mM MES, pH 6.0, 2 M NaCl). Fractions (6 mL) were analysed by an analytical SDS gel electrophoresis (4-12%). The fraction containing ST6Gal-I were pooled and dialysed against buffer A + 100 mM NaCl.

Protein concentration was determined as extinction at a wave length of 280 nm (E280nm) with an extinction value of 1.802 corresponding to a protein concentration of 10 mg/ml in the solution. For the purified enzymes the specific activities were determined.

The purity of the preparations was checked by SDS gel electrophoresis. From each sample supernatant of hST6Gal-I variants Δ27, Δ48, Δ62 and Δ89 a major protein band with an apparently uniform molecular weight of about 36 kDa was obtained (see Figure 2). The observed molecular weight corresponding to this band indicates deviations from the predicted sizes of the hST6Gal-I variants in the supernatant. In the supernatant of a clone secreting a Δ89 variant, however, a further (but less abundant) protein band corresponding to a molecular weight of between 40 and 50 kDa was observed. From the uniformly obtained major band of about 36 kDa it was concluded that the secreted proteins were proteolytically cleaved after the secretion process, presumably by proteases released into the supernatant by the *Pichia* cells.

### Example 14

### Enzymatic characterization of human ST6Gal-I variants from transformed Pichia pastoris KM71H

As described in Example 13 and furthermore shown by Figure 2, several clones of the genetic constructs were expressed in *Pichia pastoris* KM71H and purified from the supernatant. Secreted human ST6Gal-I variants were analyzed by mass-spectrometry (MS) after purification. In Table 1 (below), for each enzyme sample the composition consisting of various proteolytically truncated species is given, based on the relative peak sizes obtained in the MS data. Thus, the relative abundance of a particular species of hST6Gal-I variant is indicated in Table 1. Further, based on time-of-flight data of fragments generated in the course of MS analysis, further information was gathered concerning the composition of N-teminal fragments of the human ST6Gal-I variants. Moreover, the specific activity (RFU/µg; see Example 7) was determined.

For the Δ27 variant no active enzyme was found in the supernatant; for this reason no further analysis was conducted in this case.

Several purified samples of the construct Δ62:clone 356 were found to exhibit a high specific activity. N-terminal sequences of hST6Gal-I variants present in the samples were determined. The N-terminus "LQKIWKNYLS" was found consistently in the samples; it corresponds to a Δ108 N-terminal truncation variant of human ST6Gal-I.

Another preparation was obtained from the supernatant of Δ62:clone 356 from a separate cultivation batch; it was found to comprise a mixture of about 75% of Δ114 hST6Gal-I and about 25% of Δ112 hST6Gal-I. It showed a specific activity which was about 10% of the specific activity determined for the Δ108 hST6Gal-I variant. From this relatively low specific activity it was concluded that a deletion of 112 amino acid residues or more reduces significantly the activity of the hST6Gal-I truncation variant enzyme. An enzyme preparation consisting mainly of Δ114 hST6Gal-I was found to have a very much reduced activity which nevertheless was measurable. However, measurable activity might be attributable to small quantities of a larger truncation variant which nevertheless escaped detection.

**Table 1:**

| Analytical data of recombinantly expressed truncated variants Δ48, Δ62 and Δ108 of human ST6Gal-I isolated from transformed *Pichia pastoris* KM71H supernatants. | | | | |
|---|---|---|---|---|
| Expression construct: "clone" (*P. pastoris*) | N-terminal sequence, deduced from MS (TOF) data | truncation from N-terminus | relative abundance (percentage) as estimated from peaks of the MS spectrum | specific enzyme activity [RFU/µg] of total sample (all variant species together) |
| Δ62:clone 356 ["batch 1"] | LQKIWKNYLS... | Δ108 | ≈50 | 185.9 |
| | NYLS... | Δ114 | ≈20 | |
| | IWKNYLS ... | Δ111 | ≈15 | |
| | WKNYLS... | Δ112 | ≈10 | |
| Δ62:clone 356 ["batch 2"] | NYLS... | Δ114 | >70 | 70 |
| | WKNYLS... | Δ112 | ≈25 | |
| Δ62:clone 356 ["batch 3"] | NYLS... | Δ114 | >95 | 27.8 |
| Δ62:clone 356 ["batch 4"] | NYLS... | Δ114 | ≈75 | n.d. |
| | LQKIWKNYLS... | Δ108 | ≈25 | |
| Δ62:clone 356 ["batch 5"] | LQKIWKNYLS... | Δ108 | >95 | 663.5 |
| Δ62:clone 356 ["batch 6"] | NYLS... | Δ114 | ≈70 | 208 |
| | LQKIWKNYLS... | Δ108 | ≈30 | |
| Δ62:clone 356 ["batch 7"] | LQKIWKNYLS... | Δ108 | >95 | 689.1 |
| Δ48:clone 9 ["batch 8"] | NYLS... | Δ114 | >95 | n.d. |
| Δ108 ["batch 9"] | NYLS... | Δ114 | >95 | n.d. |

| | | | | |
|---|---|---|---|---|
| n.d. = not determined | | | | |

### Example 15

### Purification of the Δ108 N-terminal truncation variant of human ST6Gal-I from supernatants of transformed HEK cells

HEK cells were transformed as described in Example 6. The expression construct was prepared as described in Example 5. The particular hST6Gal-I coding sequence was a nucleotide sequence encoding the Δ108 hST6Gal-I N-terminal truncation variant, the expressed construct therefore was Epo-APPR-Δ108-hST6Gal-I.

From supernatants of HEK cell fermentations of the enzyme variant was purified using a simplified purification protocol. In a first step, 0.1 liter of culture supernatant was filtrated (0.2 µm), the solution was dialysed against buffer A (20 mM potassium phosphate, pH 6.5). The dialysate was loaded onto a S-Sepharose™ ff (Fast Flow) column (1.6 cm x 2 cm) equilibrated with buffer A. After washing with 100 mL buffer A, the enzyme was eluted with a linear gradient of 10 mL buffer A and 10 mL of buffer A + 200 mM NaCl, followed by a wash step using 48 mL of buffer A + 200 mM NaCl. Fractions (4 mL) were analysed by an analytical SDS gel electrophoresis. Fractions containing the enzyme were pooled and dialyzed against storage buffer (20 mM potassium phosphate, 100 mM sodium chloride, pH 6.5). Protein concentration was determined at a wave length of 280 nm using a molar extinction coefficient of 1.871. Mass spectrometric analysis of the recombinant protein secreted from the HEK cells transformed with the Epo-APPR-Δ108-hST6Gal-I expression construct confirmed the N-terminal sequence "APPR", thus indicating the expected cleavage of the EPO signal sequence by the signal peptidase. For the recombinant human Δ108 hST6Gal-I variant from HEK cells a specific activity of >600 RFU/µg was determined.

## Claims

1. A variant mammalian glycosyltransferase, wherein the polypeptide of the variant comprises an N-terminally truncated amino acid sequence of the wild-type mammalian glycosyltransferase, and wherein the polypeptide of the variant consists of the amino acid sequence from position 109 to position 406 of SEQ ID NO:1.

2. The variant according to claim 1, wherein the N-terminus or C-terminus of the polypeptide of the variant is fused to an affinity tag.

3. The variant according to claim 2, wherein a peptidase cleavage site is located between the affinity tag and the N-terminus or C-terminus of the polypeptide of the variant.

4. A nucleotide sequence encoding the polypeptide of a variant mammalian glycosyltransferase according to any of the claims 1 to 3.

5. An expression vector comprising a target gene operably linked to sequences facilitating expression of the target gene in a host organism transformed with the expression vector, wherein the target gene comprises a nucleotide sequence according to claim 4.

6. A transformed host organism, wherein the host organism is transformed with an expression vector according to claim 5.

7. A method to recombinantly produce a variant mammalian glycosyltransferase, the method comprising the step of expressing in a transformed host organism a nucleotide sequence encoding the variant mammalian glycosyltransferase according to any of the claims 1 to 3, wherein a polypeptide is formed, thereby producing the variant mammalian glycosyltransferase.

8. The method according to claim 7, wherein the produced variant mammalian glycosyltransferase is secreted from the host organism.

9. The method according to any of the claims 7 and 8, wherein the host organism is a eukaryotic cell.

10. The method according to any of the claims 7 to 9, wherein the variant mammalian glycosyltransferase is purified.

11. A variant mammalian glycosyltransferase, wherein the polypeptide of the variant consists of the amino acid sequence from position 109 to position 406 of SEQ ID NO:1, obtained by a method according to any of the claims 7 to 10.

12. Use of a variant mammalian glycosyltransferase according to any of the claims 1 to 3, or a variant of human β-galactoside-α-2,6-sialyltransferase I obtained by a method according to any of the claims 7 to 10 for transferring a 5-N-acetylneuraminic acid residue from the donor compound cytidine-5'-monophospho-N-acetylneuraminic acid, or from a functional equivalent thereof, to an acceptor, the acceptor being terminal β-D-galactosyl-1,4-N-acetyl-β-D-glucosamine in a glycan moiety of a monoclonal antibody.

## Patentansprüche

1. Säuger-Glykosyltransferase-Variante, wobei das Polypeptid der Variante eine N-terminal verkürzte Aminosäuresequenz der Wildtyp-Säuger-Glykosyltransferase umfasst, und wobei das Polypeptid der Variante aus der Aminosäuresequenz von Position 109 bis Position 406 von SEQ ID NO:1 besteht.

2. Variante nach Anspruch 1, wobei der N-Terminus oder C-Terminus des Polypeptids der Variante an eine Affinitäts-Markierung fusioniert ist.

3. Variante nach Anspruch 2, wobei sich eine Peptidase-Spaltstelle zwischen der Affinitäts-Markierung und dem N-Terminus oder C-Terminus des Polypeptids der Variante befindet.

4. Nukleotidsequenz, die das Polypeptid einer Säuger-Glykosyltransferase-Variante nach einem der Ansprüche 1 bis 3 codiert.

5. Expressionsvektor, der ein Ziel-Gen umfasst, das funktionell mit Sequenzen verknüpft ist, die die Expression des Ziel-Gens in einem Wirtsorganismus erleichtern, der mit dem Expressionsvektor transformiert wurde, wobei das Ziel-Gen eine Nukleotidsequenz nach Anspruch 4 umfasst.

6. Transformierter Wirtsorganismus, wobei der Wirtsorganismus mit einem Expressionsvektor nach Anspruch 5 transformiert ist.

7. Verfahren zum rekombinanten Produzieren einer Säuger-Glykosyltransferase-Variante, wobei das Verfahren den Schritt des Exprimierens einer Nukleotidsequenz, die die Säuger-Glykosyltransferase-Variante nach einem der Ansprüche 1 bis 3 codiert, in einem transformierten Wirtsorganismus umfasst, wobei ein Polypeptid gebildet wird, wodurch die Säuger-Glykosyltransferase-Variante produziert wird.

8. Verfahren nach Anspruch 7, wobei die produzierte Säuger-Glykosyltransferase-Variante aus dem Wirtsorganismus sekretiert wird.

9. Verfahren nach einem der Ansprüche 7 und 8, wobei der Wirtsorganismus eine eukaryotische Zelle ist.

10. Verfahren nach einem der Ansprüche 7 bis 9, wobei die Säuger-Glykosyltransferase-Variante gereinigt wird.

11. Säuger-Glykosyltransferase-Variante, wobei das Polypeptid der Variante aus der Aminosäuresequenz von Position 109 bis Position 406 von SEQ ID NO:1 besteht, die durch ein Verfahren nach einem der Ansprüche 7 bis 10 erhalten wurde.

12. Verwendung einer Säuger-Glykosyltransferase-Variante nach einem der Ansprüche 1 bis 3 oder einer Variante von humaner β-Galaktosid-α-2,6-sialyltransferase I, die durch ein Verfahren nach einem der Ansprüche 7 bis 10 erhalten wurde, zum Übertragen eines 5-N-Acetylneuraminsäurerestes aus der Donorverbindung Cytidin-5'-monophospho-N-acetylneuraminsäure oder aus einem funktionellen Äquivalent davon auf einen Akzeptor, wobei der Akzeptor terminales β-D-Galaktosyl-1,4-N-acetyl-β-D-glukosamin in einer Glykaneinheit eines monoklonalen Antikörpers ist.

## Revendications

1. Variant de glycosyltransférase de mammifère, dans lequel le polypeptide du variant comprend une séquence d'acides aminés tronquée à l'extrémité N-terminale de la glycosyltransférase de mammifère de type sauvage, et dans lequel le polypeptide du variant consiste en la séquence d'acides aminés de la position 109 à la position 406 de la SEQ ID NO : 1.

2. Variant selon la revendication 1, dans lequel l'extrémité N-terminale ou C-terminale du polypeptide du variant est fusionnée à un marqueur d'affinité.

3. Variant selon la revendication 2, dans lequel un site de coupure de peptidase est situé entre le marqueur d'affinité et l'extrémité N-terminale ou C-terminale du polypeptide du variant.

4. Séquence nucléotidique codant pour le polypeptide d'un variant de glycosyltransférase de mammifère selon l'une quelconque des revendications 1 à 3.

5. Vecteur d'expression comprenant un gène cible lié de manière fonctionnelle à des séquences facilitant l'expression du gène cible dans un organisme hôte transformé avec le vecteur d'expression, dans lequel le gène cible comprend une séquence nucléotidique selon la revendication 4.

6. Organisme hôte transformé, dans lequel l'organisme hôte est transformé avec un vecteur d'expression selon la revendication 5.

7. Procédé de production par recombinaison d'un variant de glycosyltransférase de mammifère, le procédé comprenant l'étape d'expression dans un organisme hôte transformé d'une séquence nucléotidique codant pour le variant de glycosyltransférase de mammifère selon l'une quelconque des revendications 1 à 3, dans lequel un polypeptide est formé, produisant ainsi le variant de glycosyltransférase de mammifère.

8. Procédé selon la revendication 7, dans lequel le variant de glycosyltransférase de mammifère produit est sécrété de l'organisme hôte.

9. Procédé selon l'une quelconque des revendications 7 et 8, dans lequel l'organisme hôte

10. Procédé selon l'une quelconque des revendications 7 à 9, dans lequel le variant de glycosyltransférase de mammifère est purifié.

11. Variant de glycosyltransférase de mammifère, dans lequel le polypeptide du variant consiste en la séquence d'acides aminés de la position 109 à la position 406 de la SEQ ID NO : 1, obtenu par un procédé selon l'une quelconque des revendications 7 à 10.

12. Utilisation d'un variant de glycosyltransférase de mammifère selon l'une quelconque des revendications 1 à 3, ou d'un variant de la β-galactoside-α-2,6-sialyltransférase I humaine obtenu par un procédé selon l'une quelconque des revendications 7 à 10 pour le transfert d'un résidu acide 5-N-acétylneuraminique du composé donneur acide cytidine-5'-monophospho-N-acétylneuraminique, ou d'un équivalent fonctionnel de celui-ci, à un accepteur, l'accepteur étant la β-D-galactosyl-1,4-N-acétyl-β-D-glucosamine terminale dans une fraction glycane d'un anticorps monoclonal.
